# EUROPEAN PATENT APPLICATION

(11) **EP 1 801 116 A1**
(43) Date of publication of application: **27.06.2007**
(21) Application number: 06125762.2
(22) Date of filing: 11.12.2006
(51) Int. Cl.: C07K 14/18, C12N 15/86, G01N 33/576

(54) **HCV replicon shuttle vectors**

(30) Priority: 21.12.2005 US 752866 P
(71) Applicant: F. HOFFMANN-LA ROCHE AG, 4070 Basel (CH)
(72) Inventor: Dietrich, Paul Shartzer, Palo Alto, CA 94303 (US); Kosaka, Alan H., Half Moon Bay, CA 94019 (US); Le Pogam, Sophie, Mountain View, CA 94040 (US); Najera, Isabel, Portola Valley, CA 94028 (US)

(57) **Abstract**

The present invention provides for novel HCV replicon shuttle vectors useful for cloning in HCV polynucleotide sequences from samples of HCV-infected patients and testing the resulting replicons for drug susceptibility.

## Description

This invention pertains to novel HCV replicon shuttle vectors which are useful for screening, testing and evaluating HCV polymerase inhibitors.

Hepatitis C virus is a major health problem and the leading cause of chronic liver disease throughout the world. (Boyer, N. et al. J. Hepatol. 2000 32:98-112). Patients infected with HCV are at risk of developing cirrhosis of the liver and subsequent hepatocellular carcinoma and hence HCV is the major indication for liver transplantation.

According to the World Health Organization, there are more than 200 million infected individuals worldwide, with at least 3 to 4 million people being infected each year. Once infected, about 20% of people clear the virus, but the rest can harbor HCV the rest of their lives. Ten to twenty percent of chronically infected individuals eventually develop liver-destroying cirrhosis or cancer. The viral disease is transmitted parenterally by contaminated blood and blood products, contaminated needles, or sexually and vertically from infected mothers or carrier mothers to their offspring. Current treatments for HCV infection, which are restricted to immunotherapy with recombinant interferon-□ alone or in combination with the nucleoside analog ribavirin, are of limited clinical benefit particularly for genotype 1. There is an urgent need for improved therapeutic agents that effectively combat chronic HCV infection

HCV has been classified as a member of the virus family Flaviviridae that includes the genera *flaviviruses, pestiviruses,* and *hepaciviruses* which includes hepatitis C viruses (Rice, C. M., Flaviviridae: The viruses and their replication, in: Fields Virology, Editors: Fields, B. N., Knipe, D. M., and Howley, P. M., Lippincott-Raven Publishers, Philadelphia, Pa., Chapter 30, 931-959, 1996). HCV is an enveloped virus containing a positive-sense single-stranded RNA genome of approximately 9.4 kb. The viral genome consists of a 5'-untranslated region (UTR), a long open reading frame encoding a polyprotein precursor of-approximately 3011 amino acids, and a short 3' UTR. The 5' UTR is the most highly conserved part of the HCV genome and is important for the initiation and control of polyprotein translation.

Genetic analysis of HCV has identified six main genotypes showing a >30% divergence in the DNA sequence. Each genotype contains a series of more closely related subtypes which show a 20-25 % divergence in nucleotide sequences (Simmonds, P. 2004 J. Gen. Virol. 85:3173-88) . More than 30 subtypes have been distinguished. In the US approximately 70% of infected individuals have type 1a and 1b infection. Type 1b is the most prevalent subtype in Asia. (X. Forns and J. Bukh, Clinics in Liver Disease 1999 3:693-716; J. Bukh et al., Semin. Liv. Dis. 1995 15:41-63). Unfortunately Type 1 infections are less responsive to the current therapy than either type 2 or 3 genotypes (N. N. Zein, Clin. Microbiol. Rev., 2000 13:223-235).

The genetic organization and polyprotein processing of the nonstructural protein portion of the ORF of pestiviruses and hepaciviruses is very similar. These positive stranded RNA viruses possess a single large open reading frame (ORF) encoding all the viral proteins necessary for virus replication. These proteins are expressed as a polyprotein that is co- and post-translationally processed by both cellular and virus-encoded proteinases to yield the mature viral proteins. The viral proteins responsible for the replication of the viral genome RNA are located towards the carboxy-terminal. Two-thirds of the ORF are termed nonstructural (NS) proteins. For both the pestiviruses and hepaciviruses, the mature nonstructural (NS) proteins, in sequential order from the amino-terminus of the nonstructural protein coding region to the carboxy-terminus of the ORF, consist of p7, NS2, NS3, NS4A, NS4B, NS5A, and NS5B.

The NS proteins of pestiviruses and hepaciviruses share sequence domains that are characteristic of specific protein functions. For example, the NS3 proteins of viruses in both groups possess amino acid sequence motifs characteristic of serine proteinases and of helicases (Gorbalenya et al. Nature 1988 333:22; Bazan and Fletterick Virology 1989 171:637-639; Gorbalenya et al. Nucleic Acid Res. 1989 17.3889-3897). Similarly, the NS5B proteins of pestiviruses and hepaciviruses have the motifs characteristic of RNA-directed RNA polymerases (Koonin, E. V. and Dolja, V. V. Crit. Rev. Biochem. Molec. Biol. 1993 28:375-430) .

The actual roles and functions of the NS proteins of pestiviruses and hepaciviruses in the lifecycle of the viruses are directly analogous. In both cases, the NS3 serine proteinase is responsible for all proteolytic processing of polyprotein precursors downstream of its position in the ORF (Wiskerchen and Collett Virology 1991 184:341-350; Bartenschlager et al. J. Virol. 1993 67:3835-3844; Eckart et al. Biochem. Biophys. Res. Comm. 1993 192:399-406; Grakoui et al. J. Virol. 1993 67:2832-2843; Grakoui et al. Proc. Natl. Acad. Sci. USA 1993 90:10583-10587; Ilijikata et al. J. Virol. 1993 67:4665-4675; Tome et al. J Virol. 1993 67:4017-4026). The NS4A protein, in both cases, acts as a cofactor with the NS3 serine protease (Bartenschlager et al. J. Virol. 1994 68:5045-5055; Failla et al. J. Virol. 1994 68: 3753-3760; Xu et al. J ViroL 1997 71:53 12-5322). The NS3 protein of both viruses also functions as a helicase (Kim et al. Biochem. Biophys. Res. Comm. 1995 215: 160-166; Jin and Peterson Arch. Biochem. Biophys. 1995, 323:47-53; Warrener and Collett J. Virol. 1995 69:1720-1726). Finally, the NS5B proteins of pestiviruses and hepaciviruses have the predicted RNA-dependent RNA polymerase activity (Behrens et al. EMBO 1996 15:12-22; Lechmann et al. J. Virol. 1997 71:8416-8428; Yuan et al. Biochem. Biophys. Res. Comm. 1997 232:231-235; Hagedorn, PCT WO 97/12033; Zhong et al. J. Virol. 1998 72:9365-9369).

Currently there are a limited number of approved therapies are currently available for the treatment of HCV infection. New and existing therapeutic approaches to treating HCV and inhibition of HCV NS5B polymerase have been reviewed: R. G. Gish, Sem. Liver. Dis., 1999 19:5; Di Besceglie, A. M. and Bacon, B. R., Scientific American, October: 1999 80-85; G. Lake-Bakaar, Current and Future Therapy for Chronic Hepatitis C Virus Liver Disease, Curr. Drug Targ. Infect Dis. 2003 3(3):247-253; P. Hoffmann et al., Recent patents on experimental therapy for hepatitis C virus infection (1999-2002), Exp. Opin. Ther. Patents 2003 13(11):1707-1723; F. F. Poordad et al. Developments in Hepatitis C therapy during 2000-2002, Exp. Opin. Emerging Drugs 2003 8(1):9-25; M. P. Walker et al., Promising Candidates for the treatment of chronic hepatitis C, Exp. Opin. Investig. Drugs 2003 12(8):1269-1280; S.-L. Tan et al., Hepatitis C Therapeutics: Current Status and Emerging Strategies, Nature Rev. Drug Discov. 2002 1:867-881; R. De Francesco et al. Approaching a new era for hepatitis C virus therapy: inhibitors of the NS3-4A serine protease and the NS5B RNA-dependent RNA polymerase, Antiviral Res. 2003 58:1-16; Q. M. Wang et al. Hepatitis C virus encoded proteins: targets for antiviral therapy, Drugs of the Future 2000 25(9):933-8-944; J. A. Wu and Z. Hong, Targeting NS5B-Dependent RNA Polymerase for Anti-HCV Chemotherapy Cur. Drug Targ.-Inf. Dis. 2003 3:207-219.

Despite advances in understanding the genomic organization of the virus and the functions of viral proteins, fundamental aspects of HCV replication and pathogenesis remain unknown. A major challenge in gaining experimental access to HCV replication is the lack of an efficient cell culture system that allows production of infectious virus particles. Although infection of primary cell cultures and certain human cell lines has been reported, the amounts of virus produced in those systems and the levels of HCV replication have been too low to permit detailed analyses.

The construction of selectable subgenomic HCV RNAs that replicate with minimal efficiency in the human hepatoma cell line Huh-7 has been reported. Lohman et al. reported the construction of a replicon (I₃₇₇/NS3-3') derived from a cloned full-length HCV consensus genome (genotype 1b) by deleting the C-p7 or C-NS2 region of the protein- coding region (Lohman et al., Science 1999 285: 110-113). The replicon contained the following elements: (i) the HCV 5'-UTR fused to 12 amino acids of the capsid encoding region; (ii) the neomycin phosphotransferace gene (NPTII); (iii) the IRES from encephalomyocarditis virus (EMCV), inserted downstream of the NPTII gene and which directs translation of HCV proteins NS2 or NS3 to NS5B; and (iv) the 3'-UTR. After transfection of Huh-7 cells, only those cells supporting HCV RNArepfication expressed the NPTII protein and developed resistance against the drug G418. While the cell lines derived from such G418 resistant colonies contained substantial levels of replicon RNAs and viral proteins, only 1 in 10⁶ transfected Huh-7 cells supported HCV replication.

Similar selectable HCV replicons were constructed based on an HCV-H genotype la infectious clone (Blight et al., Science 2000 290:1972-74). The HCV-H derived replicons were unable to establish efficient HCV replication, suggesting that the earlier-constructed replicons of Lohmann (1999), supra, were dependent on the particular genotype 1 b consensus cDNA clone used in those experiments. Blight et al. (2000), supra, reproduced the construction of the replicon made by Lohmann et al. (1999), supra, by carrying out a PCR- based gene assembly procedure and obtained G418-resistant Huh-7 cell colonies. Independent G418-resistant cell clones were sequenced to determine whether high-level HCV replication required adaptation of the replicon to the host cell. Multiple independent adaptive mutations that cluster in the HCV nonstructural protein NS5A were identified. The mutations conferred increased replicative ability in vitro, with transduction efficiency ranging from 0.2 to 10% of transfected cells as compared to earlier- constructed replicons in the art, e.g., the I₃₇₇/NS3-3' replicon had a 0.0001% transduction efficiency.

The present invention features the development of a novel HCV replicon shuttle vector in which unique restriction enzyme sites are introduced at the 5' and 3' ends of the NS5B gene such that NS5B sequences derived from the samples of HCV-infected patients can be cloned in the shuttle vector and the resulting replicons be evaluated for replication fitness and susceptibility to HCV polymerase inhibitors. Since an individual HCV-infected patient typically contains a genetically diverse virus population due to the high error rate of the NS5B RNA polymerase, the use of the shuttle vector of the present invention would allow the characterization of specific patient-derived NS5B variants and the sensitivity or resistance of these variants to drug treatment.

Accordingly, the present invention provides an HCV replicon shuttle vector comprising an HCV polynucleotide sequence that comprises, in order, a polynucleotide sequence encoding a NS3 protein, a polynucleotide sequence encoding a NS4A protein, a polynucleotide sequence encoding a NS4B protein, a polynucleotide sequence encoding a NS5A protein, a restriction enzyme sequence that recognizes AsiSI or SnaBI placed between 1 nucleotide and 20 nucleotides 5' from a polynucleotide sequence encoding a NS5B protein, a polynucleotide sequence encoding a NS5B protein, and a restriction enzyme sequence that recognizes SacII or RsrII placed between 1 nucleotide and 20 nucleotides 3' from a polynucleotide sequence encoding a NS5B protein. In one embodiment of the invention, the HCV polynucleotide sequence is derived from HCV genotype-1a or genotype-1b. In another embodiment of the invention, the polynucleotide sequence encoding the NS5B protein has been modified such that the NS5B protein is non-functional. In still another embodiment of the invention, the HCV replicon shuttle vector comprises an HCV polynucleotide sequence selected from SEQ ID NO:3, SEQ ID NO:4, SEQ ID NO:5, SEQ ID NO:6, SEQ ID NO:7, or SEQ ID NO:8.

A futher embodiment of the present invention provides a method for assessing the effectiveness of an HCV polymerase inhibitor to control an HCV infection in a subject comprising the steps of providing a sample from the subject infected with HCV, PCR-amplifying polynucleotide sequences encoding the NS5B protein from a plurarity of HCV quasispecies present in the sample with the use of a sense-strand primer which comprises a restriction enzyme sequence that recognizes AsiSI or SnaBI, and an anti-sense strand primer which comprises a restriction enzyme sequence that recognizes SacII or RsrII, cloning said PCR-amplifed polynucleotide sequences into an HCV replicon shuttle vector to produce chimeric HCV replicon plasmids, linearizing said chimeric HCV replicon plasmids and subjecting said linearized plasmids to *in vitro* transcription to produce chimeric HCV replicon RNAs, and transfecting a Huh7 cell line with said HCV replicon RNAs and measuring replication level of said HCV replicon RNAs in the presence or absence of the HCV polymerase inhibitor.

A still further embodiment of the present invention provides a method for assessing the effectiveness of an HCV polymerase inhibitor to control an HCV infection in a subject comprising the steps of providing a sample from the subject infected with HCV, PCR-amplifying polynucleotide sequences encoding the NS5B protein from a plurarity of HCV quasispecies present in the sample with the use of a sense-strand primer which comprises a restriction enzyme sequence that recognizes AsiSI or SnaBI, and an anti-sense strand primer which comprises a restriction enzyme sequence that recognizes SacII or RsrII, cloning said PCR-amplifed polynucleotide sequences into an HCV replicon shuttle vector to produce chimeric HCV replicon plasmids, transforming said plasmids into cells to generate a plurarity of colonies of transformed cells, pooling said colonies and isolating chimeric HCV replicon plasmids from the pooled colonies, linearizing said chimeric HCV replicon plasmids, subjecting said linearized plasmids to *in vitro* transcription to produce chimeric HCV replicon RNAs, and transfecting Huh7 cell line with said HCV replicon RNAs and measuring replication level of said HCV replicon RNAs in the presence or absence of the HCV polymerase inhibitor.

The foregoing and other advantages and features of the invention, and the manner in which the same are accomplished, will become more readily apparent upon consideration of the following detailed description of the invention taken in conjunction with the accompanying examples, which illustrate exemplary embodiments.

Figure 1 is a schematic representation of the HCV replicon shuttle vector and the positions where the restriction enzyme sites (shown as AsiSI and RsrII restriction sites) have been introduced to allow the cloning of patient-derived NS5B sequences into the vector.

Figure 2 provides a graphical representation of the experimental design of the cloning of the patient-derived NS5B PCR samples into the replicon shuttle vector and the testing of the resulting replicons.

Figure 3 shows the difference in sensitivity to the HCV polymerase inhibitors, thiophene-2-carboxylic acid and benzothidiazine across genetically diverse and treatment naive clinical isolates compared to the sensitivity of reference replicons (Con 1 for genotype-1b and H77 for genotype-1a).

### Definitions

The term "HCV replicon" refers to a nucleic acid from the Hepatitis C virus that is capable of directing the generation of copies of itself. As used herein, the term "replicon" includes RNA as well as DNA, and hybrids thereof. For example, double-stranded DNA versions of HCV genomes can be used to generate a single-stranded RNA transcript that constitutes an HCV replicon. The HCV replicons can include full length HCV genome or HCV subgenomic contructs also referred as a "subgenomic replicon". For example, the subgenomic replicons of HCV described herein contain most of the genes for the non-structural proteins of the virus, but are missing most of the genes coding for the structural proteins. Subgenomic replicons are capable of directing the expression of all of the viral genes necessary for the replication of the viral subgenome, replication of the subgenomic replicon, without the production of viral particles.

A basic HCV replicon is a subgenomic construct containing an HCV 5'-untranslated (UTR) region, an HCV NS3-NS5B polyprotein encoding region, and a HCV 3'-UTR. Other nucleic acid regions can be present such as those providing for HCV NS2, structural HCV protein(s) and non-HCV sequences.

The HCV 5'-UTR region provides an internal ribosome entry site (IRES) for protein translation and elements needed for replication. The HCV 5'-UTR region includes naturally occurring HCV 5'-UTR extending about 36 nucleotides into a HCV core encoding region, and functional derivatives thereof. The 5'-UTR region can be present in different locations such as site downstream from a sequence encoding a selection protein, a reporter, protein, or an HCV polyprotein.

In addition to the HCV 5'-UTR-PC region, non-HCV IRES elements can also be present in the replicon. The non-HCV IRES elements can be present in different locations including immediately upstream the region encoding for an HCV polyprotein. Examples of non-HCV IRES elements that can be used are the EMCV IRES, poliovirus IRES, and bovine viral diarrhea virus IRES.

The HCV 3'-UTR assists HCV replication. HCV 3' UTR includes naturally occurring HCV 3'-UTR and functional derivatives thereof. Naturally occurring 3'-UTRs include a poly U tract and an additional region of about 100 nucleotides.

The NS3-NSSB polyprotein encoding region provides for a polyprotein that can be processed in a cell into different proteins. Suitable NS3-NS5B polyprotein sequences that may be part of a replicon include those present in different HCV strains and functional equivalents thereof resulting in the processing of NS3-NS5B to produce a functional replication machinery. Proper processing can be measured for by assaying, for example, NS5B RNA dependent RNA polymerase.

The ability of an NS5B protein to provide RNA polymerase activity can be measured using techniques well known in the art. The NS5B protein is rendered "non-functional" when its RNA polymerase activity is virtually non-measurable and can be accomplished by "modifying" the polynucleotide sequence of the NS5B protein through, for example, the introduction of nucleotide substitutions, insertions or deletions.

A "vector" is a piece of DNA, such as a plasmid, phage or cosmid, to which another piece of DNA segment may be attached so as to bring about the replication, expression or integration of the attached DNA segment. A "shuttle vector" refers to a vector in which a DNA segment can be inserted into or excised from a vector at specific restriction enzyme sites. The segment of DNA that is inserted into shuttle vector generally encodes a polypeptide or RNA of interest and the restriction enzyme sites are designed to ensure insertion of the DNA segment in the proper reading frame for transcription and translation.

A variety of vectors can be used to express a nucleic acid molecule. Such vectors include chromosomal, episomal, and virus-derived vectors, e.g., vectors derived from bacterial plasmids, from bacteriophage, from yeast episomes, from yeast chromosomal elements, including yeast artificial chromosomes, from viruses such as baculoviruses, papovaviruses such as SV40, vaccinia viruses, adenoviruses, poxviruses, pseudorabies viruses, herpes viruses, and retroviruses. Vectors may also be derived from combinations of these sources, such as those derived from plasmid and bacteriophage genetic elements, e.g., cosmids and phagemids. Appropriate cloning and expression vectors for prokaryotic and eukaryotic hosts are described in Sambrook et al., (1989) Molecular Cloning: A Laboratory Manual. 2nd edn. Cold Spring Harbor Laboratory Press, Cold Spring Harbor, NY, USA.

A vector containing the appropriate nucleic acid molecule can be introduced into an appropriate host cell for propagation or expression using known techniques. Host cells can include bacterial cells including, but not limited to, *E. coli, Streptomyces,* and *Salmonella typhimurium,* eukaryotic cells including, but not limited to, yeast, insect cells, such as *Drosophila,* animal cells, such as Huh-7, HeLa, COS, HEK 293, MT- 2T, CEM-SS, and CHO cells, and plant cells.

Vectors generally include selectable markers that enable the selection of a subpopulation of cells that contain the recombinant vector constructs. The marker can be contained in the same vector that contains the nucleic acid molecules described herein or may be on a separate vector. Markers include tetracycline- or ampicillin-resistance genes for prokaryotic host cells and dihydrofolate reductase or neomycin resistance for eukaryotic host cells. However, any marker that provides selection for a phenotypic trait will be effective.

A "polynucleotide" or "nucleic acid molecule" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. "Polynucleotide" also embraces relatively short polynucleotides, often referred to as oligonucleotides.

In addition, the term "DNA molecule" refers only to the primary and secondary structure of the molecule, and does not limit it to any particular tertiary forms. Thus, the term includes double-stranded DNA found, inter alia, in linear DNA molecules (e.g., restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA).

An "RNA molecule" refers to the polymeric form of ribonucleotides in its either single-stranded form or a double-stranded helix form. In discussing the structure of particular RNA molecules, sequence may be described herein according to the normal convention of giving the sequence in the 5' to 3' direction.

The term "restriction enzyme sequence" refers to a specific double stranded-DNA sequence which is recognized and cut by bacterial enzymes, each of which cut double-stranded DNA at or near a specific nucleotide sequence. The restriction enzyme "AsiSI" recognizes the sequence 
5'GCGAT▼CGC 3'
3'CGC▲TAGCG 5'
 and cuts the double-stranded DNA after the

T residue on the recognition sequence (shown with ^{▼}_{▲}). The restriction enzyme "SnaBI"
recognizes the sequence 5'TAC^{▼}GTA 3' and cuts at the indicated nucleotide position.
3'ATG_{▲}CAT 5'

Similarly, "SacII" recognizes and cuts at 5'CCGC^{▼}GG 3' and "RsrII" recognizes and
cuts
3'GG_{▲}CGCC 5'
either at

5’CG▼GACCG 3’or at 5’CG▼GTCCG 3’
3’GCCTG▲GC 5’ 3’GCCAG▲GC 5’.

The term "primer" as used herein refers to an oligonucleotide, either RNA or DNA, either single-single-stranded or double-stranded, either derived from a biological system, generated by restriction enzyme digestion, or produced synthetically which, when placed in the proper environment, is able to functionally act as an initiator of template-dependent nucleic acid synthesis. When presented with an appropriate nucleic acid template, suitable nucleoside triphosphate precursors of nucleic acids, a polymerase enzyme, suitable cofactors and conditions such as a suitable temperature and pH, the primer may be extended at its 3' terminus by the addition of nucleotides by the action of a polymerase or similar activity to yield a primer extension product. The primer may vary in length depending on the particular conditions and requirement of the application. For example, in PCR reactions, the primer is typically 15-25 nucleotides or longer in length. The primer must be of sufficient complementarity to the desired template to prime the synthesis of the desired extension product, i.e. to be able to anneal with the desired template strand in a manner sufficient to provide the 3'-hydroxyl moiety of the primer in appropriate juxtaposition for use in the initiation of synthesis by a polymerase or similar enzyme. It is not required that the primer sequence represent an exact complement of the desired template. For example, a non-complementary nucleotide sequence (e.g. a restriction enzyme recognition sequence) may be attached to the 5'-end of an otherwise complementary primer. Alternatively, non-complementary bases may be interspersed within the oligonucleotide primer sequence, provided that the primer sequence has sufficient complementarity with the sequence of the desired template strand to functionally provide a template-primer complex for the synthesis of the extension product.

The term "chimeric" as used herein means a molecule of DNA that has resulted from DNA from two or more different sources that have been fused or spliced together.

As used herein, the term "quasispecies" means a collection of microvariants of a predominant HCV genome sequence (i.e. genotype), said microvariants being formed in a single infected subject or even in a single cell clone or even in a single cell clone as a result of high mutation rate during HCV replication.

The term "subject" as used herein refers to vertebrates, particular members of the mammalian species and includes, but not limited to, rodents, rabbits, shrews, and primates, the latter including humans.

The term "sample" refers to a sample of tissue or fluid isolated from a subject, including but not limited to, for example, plasma, serum, spinal fluid, lymph fluid, the external sections of the skin, respiratory, intestinal and genitourinary tracts, tears, saliva, milk, blood cells, tumors, organs, and also samples of in vitro cell culture constituents (including but not limited to, conditioned medium resulting from the growth of cultured cells, putatively viral infected cells, recombinant cells, and cell components).

A cell has been "transformed" or "transfected" by exogenous or heterologous DNA or RNA when such DNA or RNAhas been introduced inside the cell. The transforming or transfecting DNA or RNA may or may not be integrated (covalently linked) into chromosomal DNA making up the genome of the cell. For example, in prokaryotes, yeast, and mammalian cells, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. In the case of an HCV replicon that transforms a mammalian cell as described in the present invention, the RNA molecule, e.g., an HCV RNA molecule, has the ability to replicate semi-autonomously. Huh-7 cells carrying the HCV replicons are detected either by the presence of a selection marker or a reporter gene present on the replicon.

A "clone" refers to a population of cells derived from a single cell or common ancestor generally by the process of mitosis.

### EXAMPLES

The following preparations and examples are given to enable those skilled in the art to more clearly understand and to practice the present invention. They should not be considered as limiting the scope of the invention, but merely as being illustrative and representative thereof.

### Example 1

### Construction of Plasmids

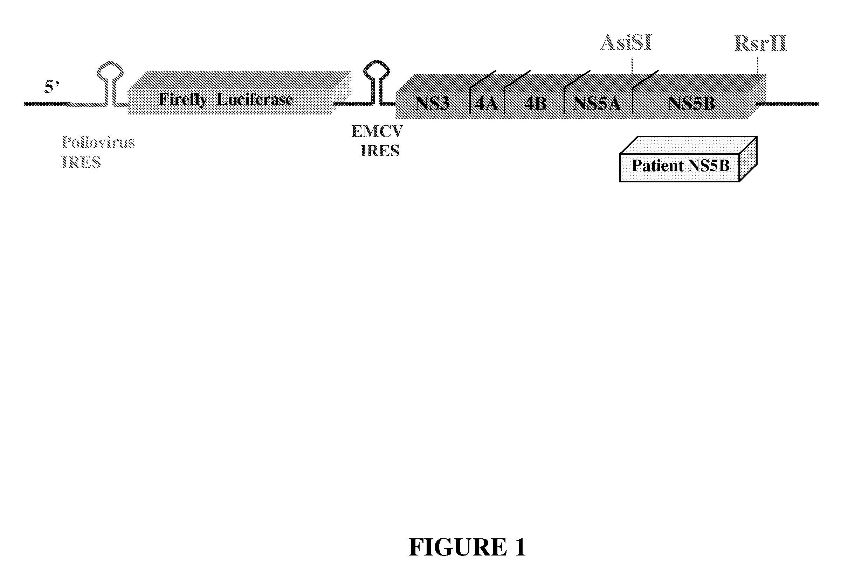

The transient HCV genotype-1b Con1 replicon vector rep PI-luc/ET, shown in Figure 1, was obtained from R. Bartenschlager and contains the HCV polynucleotide sequence from the 5'-UTR, NS3 through NS5B proteins and the 3'-UTR disclosed in GenBank Accession Number AJ238799. It also includes the poliovirus internal ribosome entry site (IRES), which controls the translation of a firefly luciferase gene. Downstream of the firefly luciferase gene, the IRES from the encephalomyocarditis virus (EMCV) controls the translation of the HCV non-structural genes (NS3, NS4A, NS4B, NS5A and NS5B).

The transient replicon repPI-luc/ET vector was modified to replace the pBR322 backbone with the pUC18 backbone to generate replicon pPI-luc/ET/SC (SEQ ID NO:1). In test assays, pPI-luc/ET/SC replicated with similar levels to rep PI-luc/ET (see Table 1).

The transient HCV genotype-1a H77 replicon vector pSS-1 was generated by ligating a SpeI - BsrGI fragment from pPI-luc/ET/SC which contains nucleotide sequence from the pUC18 backbone, 5'-UTR, poliovirus IRES, firefly luciferase, EMCV IRES and 75 amino acids from the 5'-end of the NS3 gene (genotype 1b-Con1) with a BsrGI - SpeI fragment from HCV genotype 1a H77 (Yanagi et al. Proc. Natl. Acad. Sci. U.S.A. 1997 94:8738-8743; GenBank Accession Number AF011751) which contains the remainder of the NS3 gene through the 3'-UTR, and also the S2204I adaptive mutation in the NS5A gene (Blight et al. 2000). The full nucleotide sequence of pSS-1 is shown in SEQ ID NO: 2.

For site-directed mutagenesis purpose, an intermediate vector (pSLP) was created and contains a fragment of either pPI-luc/ET/SC or pSS-1 which includes the 3'end region of the NS5A, the NS5B coding region and the 3'-UTR (XhoI-SpeI fragment) subcloned into the pBSKII+ vector. Mutations were introduced into the intermediate vector pSLP using Quick Change site-directed mutagenesis kit following manufacturers' instructions (Stratagene, La Jolla, CA, USA) and were confirmed by double-stranded DNA sequencing. The site-directed mutants described here were created in the intermediate vector pSLP and the mutant fragments XhoI-SpeI were then subcloned into the replicon vectors (pPI-luc/ET/SC or pSS-1).

To create the HCV replicon shuttle vectors used in the phenotypic assays, the intermediate vector pSLP was used to introduce enzyme restriction sites at the 3'end of the NS5A gene between 1 and 20 nucleotides 5' to the start of the NS5B gene, and in the 3'-UTR between 1 and 20 nucleotides 3' to the end of the NS5B gene. Several combinations of restriction sites in both positions were created in the pSLP vector and then subcloned into the replicon shuttle vectors. The pPI-luc/ET/SC vector was utilized as the template to synthesize the genotype 1b shuttle vectors,
pSC_1b_NSSB 5'AsiSI_3'RsrII (SEQ ID NO:3) and pSC_1b_NSSB_5'AsiSI_3'SacII (SEQ ID NO:4). pSS-1 was used as the template vector to synthesize the genotype 1a shuttle vector pSS-1_1a_NS5B_5'AsiSI_3'RsrII (SEQ ID NO: 7).

pSC_lb_ NSSB_5'AsiSI 3'SacII was used to create a deletion in the NS5B gene between the 2 Bg1II sites, removing a 1.044 kb fragment to generate the replicon shuttle vector pSC_1b_delNS5B_5'AsiSI_3'SacII (SEQ ID NO:5). The replication capability of the deleted replicon was abolished due to the absence of a functional NS5B protein (see Table 1).

Non-self replicating HCV replicon shuttle vectors were also constructed in which the NS5B nucleotide sequence within pSC_1b_NS5B_5'AsiSI_3'RsrII and pSS-1_1a_NS5B_5'AsiSI_3'RsrII were replaced with an approximately 1.0 kb nucleotide sequence fragment containing the lacZ promoter and the *Anemonia majano* cyan fluorescent protein (AmCyan) gene from plasmid pAmCyan (Clontech Laboratories Inc.) to generate the shuttle vectors pSC_16_5'AsiSI_lacZAmc_3'RsrII (SEQ ID NO:6) and pSS-1_1a_5' AsiSI_1acZAmC_3'RsrII (SEQ ID N0:8), respectively.

Replication capability of several of these shuttle vectors was assessed using the phenotypic assay described in Example 3 and the results are shown in Table 1.

### Example 2

### Cloning of the NS5B PCR samples amplified from infected patients into pSC_1b_NS5B_5'AsiSI_3'SacII and pSC_1b_delNS5B_5'AsiSI_3'SacII

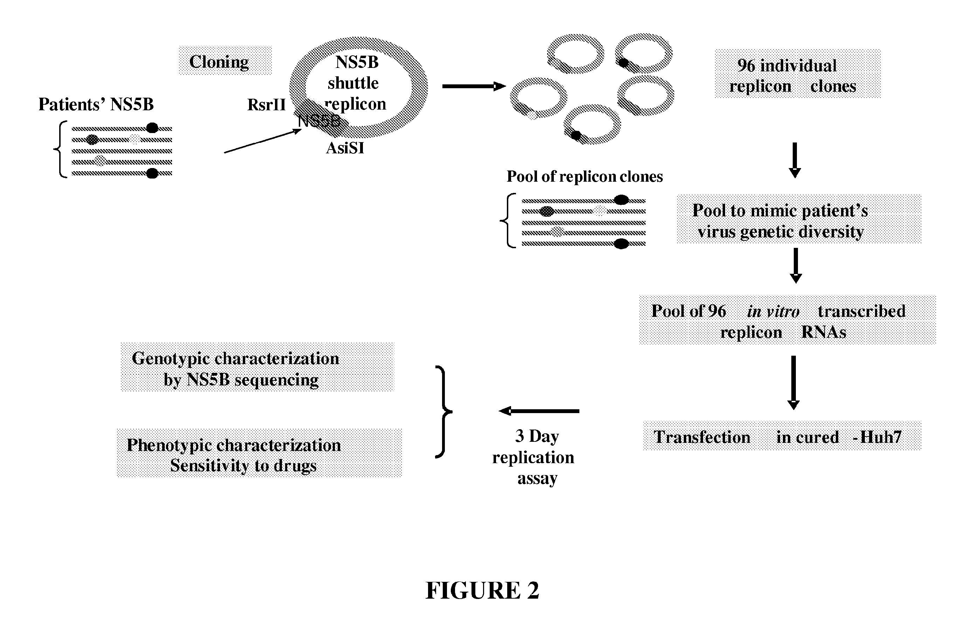

Figure 2 provides a graphical representation of the experimental design of the cloning of the patient-derived NS5B PCR samples into the replicon shuttle vector and the testing of the resulting replicons. Briefly, DNA sequences encoding the NS5B protein were PCR- generated either directly from plasma or from DNA amplified from plasma obtained from patients infected with HCV using either degenerate primers or patient specific primers wherein the sense-strand primers contain an AsiSI restriction site sequence and the antisense-strand primers contain a SacII restriction site sequence.
Sense-strand AsiSI primers used include:
5' - GGAGGCTAGTGAGGACGCGATCGCTTGCTCAATGTCCTA-3' (SEQ ID N0:9)
5' - GGAGGCTAGTGAGGACGCGATCGCTTGCTCAATGTCTTA-3' (SEQ ID NO:10)
5' - GGAGGCTAGTGAGGACGCGATCGCTTGCTCRATGTCCTA-3' (SEQ ID NO:11)
5' - GGAGGCTAGTGAGGACGCGATCGCCTGCTCAATGTCTTA-3' (SEQ ID N0:12)
5' - GGAGGCTAGTGAGGACGCGATCGCCTGCTCAATGTCCTA-3' (SEQ ID N0:13)
5' - GGAGGCTAGTGAGGACGCGATCGCTTGCTCRATGTCTTA-3' (SEQ ID N0:14)
5' - GGAGGCTAGTGAGGACGCGATCGCYTGYTCAATGTCTTA-3' (SEQ ID NO:15)
5' - GGAGGCTAGTGAGGACGCGATCGCCTGCTCGATGTCTTA-3' (SEQ ID N0:16)
5' - GGAGGCTAGTGAGGACGCGATCGCCTGTTCGATGTCCTA-3' (SEQ ID N0:17)
5' - GGAGGCTAGTGAGGACGCGATCGCCTGCTCRATGTCCTA-3' (SEQ ID N0:18)
5' - GGAGGCTAGTGAGGACGCGATCGCCTGCTCGATGTCCTA-3' (SEQ ID N0:19)

Antisense-strand SacII primers used include:
5'- GGCCTGGAGTGTTTAGCTCCGCGGTCATCGGTTGGGGTA-3' (SEQ ID N0:20)
5'- GGCCTGGAGTGTTTAGCTCCGCGGTCAYCGGTTGGGGAG-3' (SEQ ID N0:21)
5'- GGCCTGGAGTGTTTAGCTCCGCGGTCATCGGTTGGGAAG-3' (SEQ ID N0:22)
5'- GGCCTGGAGTGTTTAGCTCCGCGGTCAACGGTTGGGAAG-3' (SEQ ID N0:23)
5'- GGCCTGGAGTGTTTAGCTCCGCGGTCATCGGTTGGGAAG-3' (SEQ ID N0:24)

Accuprime Pfx (KOD polymerase, Invitrogen) was used to ensure high fidelity and robust yields. Annealing temperatures in the range of 60°-68°C were used depending upon patient sample and primer combinations. Patient NS5B DNA which were PCR amplified were then purified using Qiagen PCR Purification Columns, double digested with restriction endonucleases SgfI (Isoschizomer of AsiSI) and SacII (Promega), and gel purified using Qiagen's Gel Extraction Kit.

The shuttle replicon vector pSC_1b_ NS5B 5'AsiSI_3'SacII or the deleted shuttle replicon vector pSC_1b_delNS5B_5'AsiSI_3'SacII were prepared by double digestion with restriction endonucleases SgfI and SacII (Promega). The vectors were separated from digested insert by 1% agarose electrophoresis and gel purified using Qiagen's Gel Extraction Kit. Purified vectors were then treated with Shrimp Alkaline Phosphatase (Roche Applied Science).

Twenty-five ng of shuttle vector were ligated with the digested patient amplicons using T4 DNA ligase (Roche Applied Science) overnight at 14-16C. Vector to insert ratio of 1:2 to 1:4 were routinely used. After overnight ligation, 5 ul of the reaction were transformed into 100 ul of DH5□ Maximum Efficiency Cells (Invitrogen) and plated after 1 hour of phenotypic expression at 37C. When greater than 20% of the transformation mix was plated, sufficient number of colonies was obtained the following day.

96 individual colonies were picked to inoculate 200 ul of Terrific Broth (TB) supplemented with 50 ug/ml ampicillin. This "stock" 96-well plate was incubated overnight at 37C. The next day, this plate was used to prepare a replica plate. A 48 pin stamp was used to replica plate onto two LB plates supplemented with 100 ug/ml carbenicillin. The 96 individual 200 ul cultures were also transferred into 1.5 ml TB cultures supplemented with 50 ug/ml ampicillin to be used for DNA preparation. After an overnight incubation at 37C, the replica plate was spread with 6 mls of LB to obtain a heterogeneous pool of 96 clones, which was then used for mini-DNA preps. This DNA patient pool was then used for the subsequent Replicon Phenotypic Assay. After overnight shaking at 37C, the 96 individual 1.5 ml TB cultures were spun down, decanted and plasmid DNA was extracted using Qiagen's Qiaprep 96 Turbo Mini-DNA Kit. These individual molecular clones, which represent the composite 96 pool used for the Replicon Phenotypic Assay, were used for sequencing reactions.

Heterogeneous clone pool plasmid DNAs were submitted to sequencing to confirm the identity of patient samples and to screen for potential contaminating DNA prior to the *in vitro* transcription reaction. Individual molecular clones were submitted to sequencing for subsequent analysis in an effort to gain insight between Phenotypic Replicon Assay results and patient NS5B sequences. One-half microgram of plasmid DNA and 8 pmole of sequencing primers were routinely used.

### Example 3

*Cloning of the NS5B PCR samples amplified from infected patients into pSC_1b_NS5B_5'AsiSI_3'RsrII, pSC_1b_5'AsiSI_lacZAmC_3'RsrII, pSS-1_1a_ NSSB_ 5'AsiSI_3'Rsrll, andpSS-1_ 1a 5'AsiSl_ lacZAmC_ 3'Rsrll*

Cloning of NS5B PCR samples into the vectors which contain RsrII at the 3' end of the NS5B gene were conducted using the procedures described in Example 2 except that the following antisense-strand primers containing RsrII restriction site were used for PCR-amplifying patient NS5B:
5'-GGCCTGGAGTGTTTAGCTCGGACCGTCATCGRTTGGGGAG-3' (SEQ ID NO:25)
5'-GGCCTGGAGTGTTTAGCTCGGACCGTCACCGGTTGGGGAG-3' (SEQ ID NO:26)
5'-GGCCTGGAGTGTTTAGCTCGGACCGTCAYCGGTTGGGGAG-3' (SEQ ID NO:27)
5'-GGCCTGGAGTGTTTAGCTCGGACCGTCATCGGTTGGGAAG -3' (SEQ ID NO:28)
5'-GGCCTGGAGTGTTTAGCTCGGACCGTCAACGGTTGGGAAG-3' (SEQ ID NO:29)
5'-GGCCTGGAGTGTTTAGCTCGGACCGTCAACGGTTGGGGTA-3' (SEQ ID NO:30)
5'-GGCCTGGAGTGTTTAGCTCGGACCGTCATCGGTTGGGRAG-3' (SEQ ID NO:31)
5'-GGCCTGGAGTGTTTAGCTCGGACCGTCATCGGTTGGGGTA-3' (SEQ ID NO:32)
5'-GGCCTGGAGTGTTTAGCTCGGACCGTCATCGGTTGGGAAG-3' (SEQ ID NO:33)

### Example 4

### Replicon PhenotypicAssay

### A. Preparation of in vitro transcribed RNA

Five micrograms of DNA plasmids were linearized by Sca I restriction enzyme (Roche). After overnight digestion at 37°C, the DNA was purified using Qiagen PCR purification kit. One microgram of linearized DNA was used for the *in vitro* transcription using T7 Mega script kit following manufacturer's protocol (Ambion). After 2 hours of incubation at 37°C, DNase treatment was performed for 30 minutes at 37°C to remove the DNA template. *In vitro* transcribed RNA was then purified using NucAway Spin Column following manufacturer's protocol (Ambion).

### B. Hepatoma cell line

Cured hepatoma cell line Huh7 were cultured at 37°C in a humidified atmosphere with 5 % CO₂ in Dulbecco's Modified Eagle Medium (DMEM) supplemented with Glutamax^{TM} and 100 mg/ml sodium pyruvate (Cat# 10569-O10). The medium was further supplemented with 10 % (v/v) FBS (Cat# 16000-036) and 1 % (v/v) penicillin/streptomycin (Cat# 15140-122). All reagents were from Invitrogen/Gibco.

### C. Determination of transient replicons replication level

Four million cured Huh7 cells were transfected with 5 □g of *in vitro* transcribed RNA using electroporation. Cells were then resuspended in 7.2 ml of DMEM containing 5 % FBS and plated in 96-well plate at 50000 cells/well (in 90□1 final volume). Inhibitors were added 24 hours post-transfection in 3 fold dilutions at a final DMSO concentration of 1 % and firefly luciferase reporter signal was read 72 hours after addition of inhibitors using the Luciferase Assay system (Promega, cat # E1501). The IC₅₀ values were assessed as the inhibitor concentration at which a 50 % reduction in the level of firefly luciferase reporter was observed as compared to the level of firefly luciferase signal without the addition of compounds. Results of studies testing the inhibitory effects of the compound, 2'-C-Methyl-Cytidine, on replication of pSS-1_ La_NS5B_5'AsiSI_3'RsrII and replicons with patient-derived NS5B sequences are shown in Table 2.

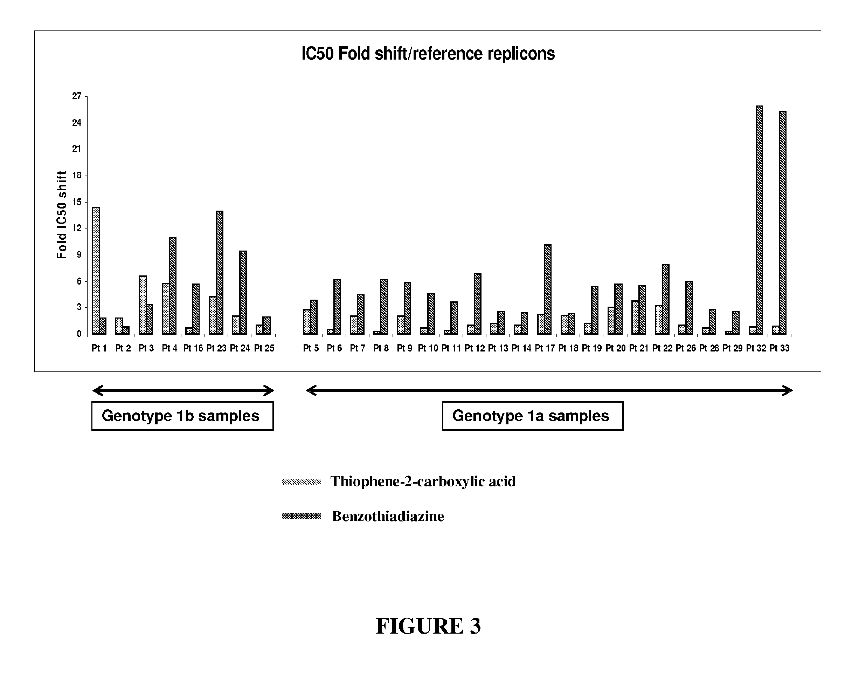

In order to test the sensitivity of HCV polymerase inhibitors across genetically diverse and treatment naive clinical isolates, the NS5B coding region from the isolates was amplified and cloned into the replicon shuttle replicons (clinical isolates from genotype-1a were cloned into pSS-1_1a_5'AsiSI_lacZAmC_3'RsrII and genotype-1b clinical isolates were cloned into pSC_1b_5'AsiSI_lacZAmC_3'RsrII). 96 molecular clones were pooled for each clinical isolate. In vitro transcription of the pooled DNA was performed. The in vitro transcribed RNA was then electroporated into Huh-7 cells as described above in the absence and presence of polymerase inhibitor(s) to determine IC50. Figure 3 shows the variable potencies exhibited by the HCV polymerase inhibitors, thiophene-2-carboxylic acid and benzothiadiazine, across the NS5B clinical isolates as compared to the reference replicons, Con1 (genotype-1b) and H77 (genotype-1a).

### D. NS5B sequencing

To determine which NS5B sequences from the patients are able to replicate during the transient assay, total cellular RNA was isolated 3 days post transient transfection using RNeasy Qiagen kit. RT-PCR and direct sequencing in the NS5B region was carried out. The NS5B nucleotide sequences from the transfected Huh7 cells were then compared to the NS5B nucleotide sequences obtained from the pool of 96 clones and to the sequences obtained by direct sequencing of HCV nucleotides in the patient sample.

**TABLE 1**

| Plasmids | Vector backbone | RLU level^{a} | Replication level^{b} |
|---|---|---|---|
| rep PI-luc/ET (WT) | rep PI-luc/ET | 106037/114192/8249 3 110649/378978 | |
| pET_1b_NS5B_ 5'SnaBI_3'SacII | rep PI-luc/ET | 259492/231992/3153 18 | WT level |
| pPI-luc/ET/SC (WT) | pPI-luc/ET/SC | 164426/319456/3311 68886337/198268 | |
| pSC_1b_NS5B_5'S naBI_3'SacII | pPI-luc/ET/SC | 354147/421737 | WT level |
| pSC_1b_NS5B_5' AsiSI_3'SacII | pPI-luc/ET/SC | 832895/170064 | WT level |
| pSC_1b_delNS5B_ 5'SnaBI_3'SacII | pPI-luc/ET/SC | Undetectable | No replication |
| pSC_1b_delNS5B_ 5'AsiSI_3'SacII | pPI-luc/ET/SC | Undetectable | No replication |

| | | | |
|---|---|---|---|
| ^{a} RLU represents level of firefly luciferase signal observed after 72 hours of replication. Numbers in each row represent different data from independent experiments ^{b}Replication level is compared to wild type vector (without introduced restriction sites). | | | |

**TABLE 2**

| NS5B Sequence | 2'-C-Me-C IC₅₀ (µM)^{a} |
|---|---|
| pSS-1_1a_NS5B_5'AsiSI_3'RsrII | 0.4 ± 0.03 |
| Clinical Isolate Pt 5 | 0.3 ± 0.05 |
| Clinical Isolate Pt 6 | 0.3 ± 0.04 |
| Clinical Isolate Pt 7 | 0.3 ± 0.05 |
| Clinical Isolate Pt 8 | 0.4 ± 0.07 |
| Clinical Isolate Pt 9 | 0.4 ± 0.06 |

| | |
|---|---|
| a Inhibition by 2'-C-Methyl-Cytidine of transient HCV replicon RNA replication containing the NS5B from genotype la- clinical isolates inserted into the shuttle vector pSS-1_1a_ NS5B_5'AsiSI_3'RsrII; mean IC50 value and standard error of mean determined from at least n=3 experiments. | |

## Claims

1. An HCV replicon shuttle vector comprising an HCV polynucleotide sequence that comprises, in order:
(a) a polynucleotide sequence encoding a NS3 protein;
(b) a polynucleotide sequence encoding a NS4A protein;
(c) a polynucleotide sequence encoding a NS4B protein;
(d) a polynucleotide sequence encoding a NS5A protein;
(e) a restriction enzyme sequence that recognizes AsiSI or SnaBI placed between 1 nucleotide and 20 nucleotides 5' from a polynucleotide sequence encoding a NS5B protein;
(f) a polynucleotide sequence encoding a NS5B protein; and
(g) a restriction enzyme sequence that recognizes SacII or RsrII placed between 1 nucleotide and 20 nucleotides 3' from a polynucleotide sequence encoding a NS5B protein.

2. The HCV replicon shuttle vector of claim 1 wherein the HCV polynucleotide sequence is derived from HCV genotype-1a or genotype-1b.

3. The HCV replicon shuttle vector of claim 1 wherein the polynucleotide sequence encoding the NS5B protein has been modified such that the NS5B protein is non-functional.

4. An HCV replicon shuttle vector comprising an HCV polynucleotide sequence selected from SEQ ID NO:3, SEQ ID NO:4, SEQ ID NO:5, SEQ ID NO:6, SEQ ID NO:7, or SEQ ID NO:8.

5. A method for assessing the effectiveness of an HCV polymerase inhibitor to control an HCV infection in a subject comprising the steps of:
(a) providing a sample from the subject infected with HCV;
(b) PCR-amplifying polynucleotide sequences encoding the NS5B protein from a plurarity of HCV quasispecies present in the sample with the use of a sense-strand primer which comprises a restriction enzyme sequence that recognizes AsiSI or SnaBI, and an anti-sense strand primer which comprises a restriction enzyme sequence that recognizes SacII or RsrII;
(c) cloning said PCR-amplifed polynucleotide sequences into an HCV replicon shuttle vector to produce chimeric HCV replicon plasmids;
(d) linearizing said chimeric HCV replicon plasmids and subjecting said linearized plasmids to *in vitro* transcription to produce chimeric HCV replicon RNAs; and
(e) transfecting a Huh7 cell line with said HCV replicon RNAs and measuring replication level of said HCV replicon RNAs in the presence or absence of the HCV polymerase inhibitor.

6. The method of claim 5 wherein the HCV replicon shuttle vector of step (c) comprises the HCV replicon shuttle vector of claim 1.

7. The method of claim 5 wherein the HCV replicon shuttle vector of step (c) comprises the HCV replicon shuttle vector of claim 2.

8. The method of claim 5 wherein the HCV replicon shuttle vector of step (c) comprises the HCV replicon shuttle vector of claim 3.

9. The method of claim 5 wherein the HCV replicon shuttle vector of step (c) comprises the HCV replicon shuttle vector of claim 4.

10. A method for assessing the effectiveness of an HCV polymerase inhibitor to control an HCV infection in a subject comprising the steps of:
(a) providing a sample from the subject infected with HCV;
(a) PCR-amplifying polynucleotide sequences encoding the NS5B protein from a plurarity of HCV quasispecies present in the sample with the use of a sense-strand primer comprising a nucleotide sequence selected from SEQ ID N0:9 thru SEQ ID NO: 19, and an anti-sense strand primer comprising a nucleotide selected from SEQ ID NO: 20 thru SEQ ID NO:33;
(b) cloning said PCR-amplifed polynucleotide sequences into an HCV replicon shuttle vector to produce chimeric HCV replicon plasmids;
(c) linearizing said chimeric HCV replicon plasmids and subjecting said linearized plasmids to *in vitro* transcription to produce chimeric HCV replicon RNAs; and
(d) transfecting Huh7 cell line with said HCV replicon RNAs and measuring replication level of said HCV replicon RNAs in the presence or absence of the HCV polymerase inhibitor.

11. The method of claim 10 wherein the HCV replicon shuttle vector of step (c) comprises the HCV replicon shuttle vector of claim 1.

12. The method of claim 10 wherein the HCV replicon shuttle vector of step (c) comprises the HCV replicon shuttle vector of claim 2.

13. The method of claim 5 wherein the HCV replicon shuttle vector of step (c) comprises the HCV replicon shuttle vector of claim 3.

14. The method of claim 5 wherein the HCV replicon shuttle vector of step (c) comprises the HCV replicon shuttle vector of claim 4.

15. A method for assessing the effectiveness of an HCV polymerase inhibitor to control an HCV infection in a subject comprising the steps of:
(a) providing a sample from the subject infected with HCV;
(b) PCR-amplifying polynucleotide sequences encoding the NS5B protein from a plurarity of HCV quasispecies present in the sample with the use of a sense-strand primer which comprises a restriction enzyme sequence that recognizes AsiSI or SnaBI, and an anti-sense strand primer which comprises a restriction enzyme sequence that recognizes SacII or RsrII;
(c) © cloning said PCR-amplifed polynucleotide sequences into an HCV replicon shuttle vector to produce chimeric HCV replicon plasmids;
(d) transforming said plasmids into cells to generate a plurarity of colonies of transformed cells;
(e) pooling said colonies and isolating chimeric HCV replicon plasmids from the pooled colonies;
(f) linearizing said chimeric HCV replicon plasmids from step (e) and subjecting said linearized plasmids to *in vitro* transcription to produce chimeric HCV replicon RNAs; and
(g) transfecting Huh7 cell line with said HCV replicon RNAs and measuring replication level of said HCV replicon RNAs in the presence or absence of the HCV polymerase inhibitor.

16. The method of claim 15 wherein the HCV replicon shuttle vector of step (c) comprises the HCV replicon shuttle vector of claim 1.

17. The method of claim 15 wherein the HCV replicon shuttle vector of step (c) comprises the HCV replicon shuttle vector of claim 2.

18. The method of claim 15 wherein the HCV replicon shuttle vector of step (c) comprises the HCV replicon shuttle vector of claim 3.

19. The method of claim 15 wherein the HCV replicon shuttle vector of step (c) comprises the HCV replicon shuttle vector of claim 4.

20. A method for assessing the effectiveness of an HCV polymerase inhibitor to control an HCV infection in a subject comprising the steps of:
(a) providing a sample from the subject infected with HCV;
(b) PCR-amplifying polynucleotide sequences encoding the NS5B protein from a plurarity of HCV quasispecies present in the sample with the use of a sense-strand primer comprising a nucleotide sequence selected from SEQ ID N0:9 thru SEQ ID NO: 19, and an anti-sense strand primer comprising a nucleotide selected from SEQ ID NO: 20 thru SEQ ID NO:33 ;
(c) cloning said PCR-amplifed polynucleotide sequences into an HCV replicon shuttle vector to produce chimeric HCV replicon plasmids;
(d) transforming said plasmids into cells to generate a plurarity of colonies of transformed cells;
(e) pooling said colonies and isolating chimeric HCV replicon plasmids from the pooled colonies;
(f) linearizing said chimeric HCV replicon plasmids from step (e) and subjecting said linearized plasmids to *in vitro* transcription to produce chimeric HCV replicon RNAs; and
(g) transfecting Huh7 cell line with said HCV replicon RNAs and measuring replication level of said HCV replicon RNAs in the presence or absence of the HCV polymerase inhibitor.

21. The method of claim 20 wherein the HCV replicon shuttle vector of step (c) comprises the HCV replicon shuttle vector of claim 1.

22. The method of claim 20 wherein the HCV replicon shuttle vector of step (c) comprises the HCV replicon shuttle vector of claim 2.

23. The method of claim 20 wherein the HCV replicon shuttle vector of step (c) comprises the HCV replicon shuttle vector of claim 3.

24. The method of claim 20 wherein the HCV replicon shuttle vector of step (c) comprises the HCV replicon shuttle vector of claim 4.


SEQUENCE LISTING

<110>  F. Hoffmann-La Roche AG

<120>  HCV Shuttle Replicon Vectors

<130>  23543

<160>  33

<170>  PatentIn version 3.2

<210>  1
<211>  11518
<212>  DNA
<213>  Artificial Sequence

<220>
<223>  Nucleotide sequence of plasmid pPI-luc/ET/SC

<400>  1
cctgcaggta atacgactca ctatagccag cccccgattg ggggcgacac tccaccatag      60

atcactcccc tgtgaggaac tactgtcttc acgcagaaag cgtctagcca tggcgttagt     120

atgagtgtcg tgcagcctcc aggacccccc ctcccgggag agccatagtg gtctgcggaa     180

ccggtgagta caccggaatt gccaggacga ccgggtcctt tcttggatca acccgctcaa     240

tgcctggaga tttgggcgtg cccccgcgag actgctagcc gagtagtgtt gggtcgcgaa     300

aggccttgtg gtactgcctg atagggtgct tgcgagtgcc ccgggaggtc tcgtagaccg     360

tgcaccgttt aaacccccgt gctgctggaa gtcgatttca ggcttagggt aaccgtggac     420

ctcgaaaaca gacgcacaaa accaagttca atagaagggg gtacaaacca gtaccaccac     480

gaacaagcac ttctgtttcc ccggtgatgt cgtatagact gcttgcgtgg ttgaaagcga     540

cggatccgtt atccgcttat gtacttcgag aagcccagta ccacctcgga atcttcgatg     600

cgttgcgctc agcactcaac cccagagtgt agcttaggct gatgagtctg gacatccctc     660

accggtgacg gtggtccagg ctgcgttggc ggcctaccta tggctaacgc catgggacgc     720

tagttgtgaa caaggtgtga agagcctatt gagctacata agaatcctcc ggcccctgaa     780

tgcggctaat cccaacctcg gagcaggtgg tcacaaacca gtgattggcc tgtcgtaacg     840

cgcaagtccg tggcggaacc gactactttg ggtgtccgtg tttcctttta ttttattgtg     900

gctgcttatg gtgacaatca cagattgtta tcataaagcg aattggattg gccatccggt     960

gaaagtgaga ctcattatct atctgtttgc tggatccgct ccattgagtg tgtttactct    1020

aagtacaatt tcaacagtta tttcaatcag acaattgtat cataatggcg ggcccagaag 1080

acgccaaaaa cataaagaaa ggcccggcgc cattctatcc tctagaggat ggaaccgctg 1140

gagagcaact gcataaggct atgaagagat acgccctggt tcctggaaca attgctttta 1200

cagatgcaca tatcgaggtg aacatcacgt acgcggaata cttcgaaatg tccgttcggt 1260

tggcagaagc tatgaaacga tatgggctga atacaaatca cagaatcgtc gtatgcagtg 1320

aaaactctct tcaattcttt atgccggtgt tgggcgcgtt atttatcgga gttgcagttg 1380

cgcccgcgaa cgacatttat aatgaacgtg aattgctcaa cagtatgaac atttcgcagc 1440

ctaccgtagt gtttgtttcc aaaaaggggt tgcaaaaaat tttgaacgtg caaaaaaaat 1500

taccaataat ccagaaaatt attatcatgg attctaaaac ggattaccag ggatttcagt 1560

cgatgtacac gttcgtcaca tctcatctac ctcccggttt taatgaatac gattttgtac 1620

cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccagg acctcgtcgg 3600

ctggcaagcg ccccccgggg cgcgttcctt gacaccatgc acctgcggca gctcggacct 3660

ttacttggtc acgaggcatg ccgatgtcat tccggtgcgc cggcggggcg acagcagggg 3720

gagcctactc tcccccaggc ccgtctccta cttgaagggc tcttcgggcg gtccactgct 3780

ctgcccctcg gggcacgctg tgggcatctt tcgggctgcc gtgtgcaccc gaggggttgc 3840

gaaggcggtg gactttgtac ccgtcgagtc tatggaaacc actatgcggt ccccggtctt 3900

cacggacaac tcgtcccctc cggccgtacc gcagacattc caggtggccc atctacacgc 3960

ccctactggt agcggcaaga gcactaaggt gccggctgcg tatgcagccc aagggtataa 4020

ggtgcttgtc ctgaacccgt ccgtcgccgc caccctaggt ttcggggcgt atatgtctaa 4080

ggcacatggt atcgacccta acatcagaat cggggtaagg accatcacca cgggtgcccc 4140

catcacgtac tccacctatg gcaagtttct tgccgacggt ggttgctctg gggcgcccta 4200

tgacatcata atatgtgatg agtgccactc aactgactcg accactatcc tgggcatcgg 4260

cacagtcctg gaccaagcgg agacggctgg agcgcgactc gtcgtgctcg ccaccgctac 4320

gcctccggga tcggtcaccg tgccacatcc aaacatcgag gaggtggctc tgtccagcac 4380

tggagaaatc cccttttatg gcaaagccat ccccatcgag accatcaagg gggggaggca 4440

cctcattttc tgccattcca agaagaaatg tgatgagctc gccgcgaagc tgtccggcct 4500

cggactcaat gctgtagcat attaccgggg ccttgatgta tccgtcatac caactagcgg 4560

agacgtcatt gtcgtagcaa cggacgctct aatgacgggc tttaccggcg atttcgactc 4620

agtgatcgac tgcaatacat gtgtcaccca gacagtcgac ttcagcctgg acccgacctt 4680

caccattgag acgacgaccg tgccacaaga cgcggtgtca cgctcgcagc ggcgaggcag 4740

gactggtagg ggcaggatgg gcatttacag gtttgtgact ccaggagaac ggccctcggg 4800

catgttcgat tcctcggttc tgtgcgagtg ctatgacgcg ggctgtgctt ggtacgagct 4860

cacgcccgcc gagacctcag ttaggttgcg ggcttaccta aacacaccag ggttgcccgt 4920

ctgccaggac catctggagt tctgggagag cgtctttaca ggcctcaccc acatagacgc 4980

ccatttcttg tcccagacta agcaggcagg agacaacttc ccctacctgg tagcatacca 5040

ggctacggtg tgcgccaggg ctcaggctcc acctccatcg tgggaccaaa tgtggaagtg 5100

tctcatacgg ctaaagccta cgctgcacgg gccaacgccc ctgctgtata ggctgggagc 5160

cgttcaaaac gaggttacta ccacacaccc cataaccaaa tacatcatgg catgcatgtc 5220

ggctgacctg gaggtcgtca cgagcacctg ggtgctggta ggcggagtcc tagcagctct 5280

ggccgcgtat tgcctgacaa caggcagcgt ggtcattgtg ggcaggatca tcttgtccgg 5340

aaagccggcc atcattcccg acagggaagt cctttaccgg gagttcgatg agatggaaga 5400

gtgcgcctca cacctccctt acatcgaaca gggaatgcag ctcgccgaac aattcaaaca 5460

gaaggcaatc gggttgctgc aaacagccac caagcaagcg gaggctgctg ctcccgtggt 5520

ggaatccaag tggcggaccc tcgaagcctt ctgggcgaag catatgtgga atttcatcag 5580

cgggatacaa tatttagcag gcttgtccac tctgcctggc aaccccgcga tagcatcact 5640

gatggcattc acagcctcta tcaccagccc gctcaccacc caacataccc tcctgtttaa 5700

catcctgggg ggatgggtgg ccgcccaact tgctcctccc agcgctgctt ctgctttcgt 5760

aggcgccggc atcgctggag cggctgttgg cagcataggc cttgggacgg tgcttgtgga 5820

tattttggca ggttatggag caggggtggc aggcgcgctc gtggccttta aggtcatgag 5880

cggcgagatg ccctccaccg aggacctggt taacctactc cctgctatcc tctcccctgg 5940

cgccctagtc gtcggggtcg tgtgcgcagc gatactgcgt cggcacgtgg gcccagggga 6000

gggggctgtg cagtggatga accggctgat agcgttcgct tcgcggggta accacgtctc 6060

ccccacgcac tatgtgcctg agagcgacgc tgcagcacgt gtcactcaga tcctctctag 6120

tcttaccatc actcagctgc tgaagaggct tcaccagtgg atcaacgagg actgctccac 6180

gccatgctcc ggctcgtggc taagagatgt ttgggattgg atatgcacgg tgttgactga 6240

tttcaagacc tggctccagt ccaagctcct gccgcgattg ccgggagtcc ccttcttctc 6300

atgtcaacgt gggtacaagg gagtctggcg gggcgacggc atcatgcaaa ccacctgccc 6360

atgtggagca cagatcaccg gacatgtgaa aaacggttcc atgaggatcg tggggcctag 6420

gacctgtagt aacacgtggc atggaacatt ccccattaac gcgtacacca cgggcccctg 6480

cacgccctcc ccggcgccaa attattctag ggcgctgtgg cgggtggctg ctgaggagta 6540

cgtggaggtt acgcgggtgg gggatttcca ctacgtgacg ggcatgacca ctgacaacgt 6600

aaagtgcccg tgtcaggttc cggcccccga attcttcaca gaagtggatg gggtgcggtt 6660

gcacaggtac gctccagcgt gcaaacccct cctacgggag gaggtcacat tcctggtcgg 6720

gctcaatcaa tacctggttg ggtcacagct cccatgcgag cccgaaccgg acgtagcagt 6780

gctcacttcc atgctcaccg acccctccca cattacggcg gagacggcta agcgtaggct 6840

ggccagggga tctcccccct ccttggccag ctcatcagct agccagctgt ctgcgccttc 6900

cttgaaggca acatgcacta cccgtcatga ctccccggac gctgacctca tcgaggccaa 6960

cctcctgtgg cggcaggaga tgggcgggaa catcacccgc gtggagtcag aaaataaggt 7020

agtaattttg gactctttcg agccgctcca agcggaggag gatgagaggg aagtatccgt 7080

tccggcggag atcctgcgga ggtccaggaa attccctcga gcgatgccca tatgggcacg 7140

cccggattac aaccctccac tgttagagtc ctggaaggac ccggactacg tccctccagt 7200

ggtacacggg tgtccattgc cgcctgccaa ggcccctccg ataccacctc cacggaggaa 7260

gaggacggtt gtcctgtcag aatctaccgt gtcttctgcc ttggcggagc tcgccacaaa 7320

gaccttcggc agctccgaat cgtcggccgt cgacagcggc acggcaacgg cctctcctga 7380

ccagccctcc gacgacggcg acgcgggatc cgacgttgag tcgtactcct ccatgccccc 7440

ccttgagggg gagccggggg atcccgatct cagcgacggg tcttggtcta ccgtaagcga 7500

ggaggctagt gaggacgtcg tctgctgctc gatgtcctac acatggacag gcgccctgat 7560

cacgccatgc gctgcggagg aaaccaagct gcccatcaat gcactgagca actctttgct 7620

ccgtcaccac aacttggtct atgctacaac atctcgcagc gcaagcctgc ggcagaagaa 7680

ggtcaccttt gacagactgc aggtcctgga cgaccactac cgggacgtgc tcaaggagat 7740

gaaggcgaag gcgtccacag ttaaggctaa acttctatcc gtggaggaag cctgtaagct 7800

gacgccccca cattcggcca gatctaaatt tggctatggg gcaaaggacg tccggaacct 7860

atccagcaag gccgttaacc acatccgctc cgtgtggaag gacttgctgg aagacactga 7920

gacaccaatt gacaccacca tcatggcaaa aaatgaggtt ttctgcgtcc aaccagagaa 7980

ggggggccgc aagccagctc gccttatcgt attcccagat ttgggggttc gtgtgtgcga 8040

gaaaatggcc ctttacgatg tggtctccac cctccctcag gccgtgatgg gctcttcata 8100

cggattccaa tactctcctg gacagcgggt cgagttcctg gtgaatgcct ggaaagcgaa 8160

gaaatgccct atgggcttcg catatgacac ccgctgtttt gactcaacgg tcactgagaa 8220

tgacatccgt gttgaggagt caatctacca atgttgtgac ttggcccccg aagccagaca 8280

ggccataagg tcgctcacag agcggcttta catcgggggc cccctgacta attctaaagg 8340

gcagaactgc ggctatcgcc ggtgccgcgc gagcggtgta ctgacgacca gctgcggtaa 8400

taccctcaca tgttacttga aggccgctgc ggcctgtcga gctgcgaagc tccaggactg 8460

cacgatgctc gtatgcggag acgaccttgt cgttatctgt gaaagcgcgg ggacccaaga 8520

ggacgaggcg agcctacggg ccttcacgga ggctatgact agatactctg ccccccctgg 8580

ggacccgccc aaaccagaat acgacttgga gttgataaca tcatgctcct ccaatgtgtc 8640

agtcgcgcac gatgcatctg gcaaaagggt gtactatctc acccgtgacc ccaccacccc 8700

ccttgcgcgg gctgcgtggg agacagctag acacactcca gtcaattcct ggctaggcaa 8760

catcatcatg tatgcgccca ccttgtgggc aaggatgatc ctgatgactc atttcttctc 8820

catccttcta gctcaggaac aacttgaaaa agccctagat tgtcagatct acggggcctg 8880

ttactccatt gagccacttg acctacctca gatcattcaa cgactccatg gccttagcgc 8940

attttcactc catagttact ctccaggtga gatcaatagg gtggcttcat gcctcaggaa 9000

acttggggta ccgcccttgc gagtctggag acatcgggcc agaagtgtcc gcgctaggct 9060

actgtcccag ggggggaggg ctgccacttg tggcaagtac ctcttcaact gggcagtaag 9120

gaccaagctc aaactcactc caatcccggc tgcgtcccag ttggatttat ccagctggtt 9180

cgttgctggt tacagcgggg gagacatata tcacagcctg tctcgtgccc gaccccgctg 9240

gttcatgtgg tgcctactcc tactttctgt aggggtaggc atctatctac tccccaaccg 9300

atgaacgggg agctaaacac tccaggccaa taggccatcc tgtttttttc cctttttttt 9360

tttctttttt tttttttttt tttttttttt ttttttttct cctttttttt tcctcttttt 9420

ttccttttct ttcctttggt ggctccatct tagccctagt cacggctagc tgtgaaaggt 9480

ccgtgagccg cttgactgca gagagtgctg atactggcct ctctgcagat caagtactac 9540

tagtagaggc ggtttgcgta ttgggcgctc ttccgcttcc tcgctcactg actcgctgcg 9600

ctcggtcgtt cggctgcggc gagcggtatc agctcactca aaggcggtaa tacggttatc 9660

cacagaatca ggggataacg caggaaagaa catgtgagca aaaggccagc aaaaggccag 9720

gaaccgtaaa aaggccgcgt tgctggcgtt tttccatagg ctccgccccc ctgacgagca 9780

tcacaaaaat cgacgctcaa gtcagaggtg gcgaaacccg acaggactat aaagatacca 9840

ggcgtttccc cctggaagct ccctcgtgcg ctctcctgtt ccgaccctgc cgcttaccgg 9900

atacctgtcc gcctttctcc cttcgggaag cgtggcgctt tctcatagct cacgctgtag 9960

gtatctcagt tcggtgtagg tcgttcgctc caagctgggc tgtgtgcacg aaccccccgt 10020

tcagcccgac cgctgcgcct tatccggtaa ctatcgtctt gagtccaacc cggtaagaca 10080

cgacttatcg ccactggcag cagccactgg taacaggatt agcagagcga ggtatgtagg 10140

cggtgctaca gagttcttga agtggtggcc taactacggc tacactagaa ggacagtatt 10200

tggtatctgc gctctgctga agccagttac cttcggaaaa agagttggta gctcttgatc 10260

cggcaaacaa accaccgctg gtagcggtgg tttttttgtt tgcaagcagc agattacgcg 10320

cagaaaaaaa ggatctcaag aagatccttt gatcttttct acggggtctg acgctcagtg 10380

gaacgaaaac tcacgttaag ggattttggt catgagatta tcaaaaagga tcttcaccta 10440

gatcctttta aattaaaaat gaagttttaa atcaatctaa agtatatatg agtaaacttg 10500

gtctgacagt taccaatgct taatcagtga ggcacctatc tcagcgatct gtctatttcg 10560

ttcatccata gttgcctgac tccccgtcgt gtagataact acgatacggg agggcttacc 10620

atctggcccc agtgctgcaa tgataccgcg agacccacgc tcaccggctc cagatttatc 10680

agcaataaac cagccagccg gaagggccga gcgcagaagt ggtcctgcaa ctttatccgc 10740

ctccatccag tctattaatt gttgccggga agctagagta agtagttcgc cagttaatag 10800

tttgcgcaac gttgttgcca ttgctacagg catcgtggtg tcacgctcgt cgtttggtat 10860

ggcttcattc agctccggtt cccaacgatc aaggcgagtt acatgatccc ccatgttgtg 10920

caaaaaagcg gttagctcct tcggtcctcc gatcgttgtc agaagtaagt tggccgcagt 10980

gttatcactc atggttatgg cagcactgca taattctctt actgtcatgc catccgtaag 11040

atgcttttct gtgactggtg agtactcaac caagtcattc tgagaatagt gtatgcggcg 11100

accgagttgc tcttgcccgg cgtcaatacg ggataatacc gcgccacata gcagaacttt 11160

aaaagtgctc atcattggaa aacgttcttc ggggcgaaaa ctctcaagga tcttaccgct 11220

gttgagatcc agttcgatgt aacccactcg tgcacccaac tgatcttcag catcttttac 11280

tttcaccagc gtttctgggt gagcaaaaac aggaaggcaa aatgccgcaa aaaagggaat 11340

aagggcgaca cggaaatgtt gaatactcat actcttcctt tttcaatatt attgaagcat 11400

ttatcagggt tattgtctca tgagcggata catatttgaa tgtatttaga aaaataaaca 11460

aataggggtt ccgcgcacat ttccccgaaa agtgccacct gacgtctaag aaaccatt 11518

<210> 2
<211> 11509
<212> DNA
<213> Artificial Sequence

<220>
<223> Nucleotide sequence of plasmid pSS-1

<400> 2
cctgcaggta atacgactca ctatagccag cccccgattg ggggcgacac tccaccatag 60

atcactcccc tgtgaggaac tactgtcttc acgcagaaag cgtctagcca tggcgttagt 120

atgagtgtcg tgcagcctcc aggacccccc ctcccgggag agccatagtg gtctgcggaa 180

ccggtgagta caccggaatt gccaggacga ccgggtcctt tcttggatca acccgctcaa 240

tgcctggaga tttgggcgtg cccccgcgag actgctagcc gagtagtgtt gggtcgcgaa 300

aggccttgtg gtactgcctg atagggtgct tgcgagtgcc ccgggaggtc tcgtagaccg 360

tgcaccgttt aaacccccgt gctgctggaa gtcgatttca ggcttagggt aaccgtggac 420

ctcgaaaaca gacgcacaaa accaagttca atagaagggg gtacaaacca gtaccaccac 480

gaacaagcac ttctgtttcc ccggtgatgt cgtatagact gcttgcgtgg ttgaaagcga 540

cggatccgtt atccgcttat gtacttcgag aagcccagta ccacctcgga atcttcgatg 600

cgttgcgctc agcactcaac cccagagtgt agcttaggct gatgagtctg gacatccctc 660

accggtgacg gtggtccagg ctgcgttggc ggcctaccta tggctaacgc catgggacgc 720

tagttgtgaa caaggtgtga agagcctatt gagctacata agaatcctcc ggcccctgaa 780

tgcggctaat cccaacctcg gagcaggtgg tcacaaacca gtgattggcc tgtcgtaacg 840

cgcaagtccg tggcggaacc gactactttg ggtgtccgtg tttcctttta ttttattgtg 900

gctgcttatg gtgacaatca cagattgtta tcataaagcg aattggattg gccatccggt 960

gaaagtgaga ctcattatct atctgtttgc tggatccgct ccattgagtg tgtttactct 1020

aagtacaatt tcaacagtta tttcaatcag acaattgtat cataatggcg ggcccagaag 1080

acgccaaaaa cataaagaaa ggcccggcgc cattctatcc tctagaggat ggaaccgctg 1140

gagagcaact gcataaggct atgaagagat acgccctggt tcctggaaca attgctttta 1200

cagatgcaca tatcgaggtg aacatcacgt acgcggaata cttcgaaatg tccgttcggt 1260

tggcagaagc tatgaaacga tatgggctga atacaaatca cagaatcgtc gtatgcagtg 1320

aaaactctct tcaattcttt atgccggtgt tgggcgcgtt atttatcgga gttgcagttg 1380

cgcccgcgaa cgacatttat aatgaacgtg aattgctcaa cagtatgaac atttcgcagc 1440

ctaccgtagt gtttgtttcc aaaaaggggt tgcaaaaaat tttgaacgtg caaaaaaaat 1500

taccaataat ccagaaaatt attatcatgg attctaaaac ggattaccag ggatttcagt 1560

cgatgtacac gttcgtcaca tctcatctac ctcccggttt taatgaatac gattttgtac 1620

cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccaag accttgtggg 3600

ctggcccgct cctcaaggtt cccgctcatt gacaccctgt acctgcggct cctcggacct 3660

ttacctggtc acgaggcacg ccgatgtcat tcccgtgcgc cggcgaggtg atagcagggg 3720

tagcctgctt tcgccccggc ccatttccta cttgaaaggc tcctcggggg gtccgctgtt 3780

gtgccccgcg ggacacgccg tgggcctatt cagggccgcg gtgtgcaccc gtggagtggc 3840

taaagcggtg gactttatcc ctgtggagaa cctagggaca accatgagat ccccggtgtt 3900

cacggacaac tcctctccac cagcagtgcc ccagagcttc caggtggccc acctgcatgc 3960

tcccaccggc agcggtaaga gcaccaaggt cccggctgcg tacgcagccc agggctacaa 4020

ggtgttggtg ctcaacccct ctgttgctgc aacgctgggc tttggtgctt acatgtccaa 4080

ggcccatggg gttgatccta atatcaggac cggggtgaga acaattacca ctggcagccc 4140

catcacgtac tccacctacg gcaagttcct tgccgacggc gggtgctcag gaggtgctta 4200

tgacataata atttgtgacg agtgccactc cacggatgcc acatccatct tgggcatcgg 4260

cactgtcctt gaccaagcag agactgcggg ggcgagactg gttgtgctcg ccactgctac 4320

ccctccgggc tccgtcactg tgtcccatcc taacatcgag gaggttgctc tgtccaccac 4380

cggagagatc cccttttacg gcaaggctat ccccctcgag gtgatcaagg ggggaagaca 4440

tctcatcttc tgccactcaa agaagaagtg cgacgagctc gccgcgaagc tggtcgcatt 4500

gggcatcaat gccgtggcct actaccgcgg tcttgacgtg tctgtcatcc cgaccagcgg 4560

cgatgttgtc gtcgtgtcga ccgatgctct catgactggc tttaccggcg acttcgactc 4620

tgtgatagac tgcaacacgt gtgtcactca gacagtcgat ttcagccttg accctacctt 4680

taccattgag acaaccacgc tcccccagga tgctgtctcc aggactcaac gccggggcag 4740

gactggcagg gggaagccag gcatctatag atttgtggca ccgggggagc gcccctccgg 4800

catgttcgac tcgtccgtcc tctgtgagtg ctatgacgcg ggctgtgctt ggtatgagct 4860

cacgcccgcc gagactacag ttaggctacg agcgtacatg aacaccccgg ggcttcccgt 4920

gtgccaggac catcttgaat tttgggaggg cgtctttacg ggcctcactc atatagatgc 4980

ccacttttta tcccagacaa agcagagtgg ggagaacttt ccttacctgg tagcgtacca 5040

agccaccgtg tgcgctaggg ctcaagcccc tcccccatcg tgggaccaga tgtggaagtg 5100

tttgatccgc cttaaaccca ccctccatgg gccaacaccc ctgctataca gactgggcgc 5160

tgttcagaat gaagtcaccc tgacgcaccc aatcaccaaa tacatcatga catgcatgtc 5220

ggccgacctg gaggtcgtca cgagcacctg ggtgctcgtt ggcggcgtcc tggctgctct 5280

ggccgcgtat tgcctgtcaa caggctgcgt ggtcatagtg ggcaggatcg tcttgtccgg 5340

gaagccggca attatacctg acagggaggt tctctaccag gagttcgatg agatggaaga 5400

gtgctctcag cacttaccgt acatcgagca agggatgatg ctcgctgagc agttcaagca 5460
gaaggccctc ggcctcctgc agaccgcgtc ccgccatgca gaggttatca cccctgctgt 5520
ccagaccaac tggcagaaac tcgaggtctt ttgggcgaag cacatgtgga atttcatcag 5580
tgggatacaa tacttggcgg gcctgtcaac gctgcctggt aaccccgcca ttgcttcatt 5640
gatggctttt acagctgccg tcaccagccc actaaccact ggccaaaccc tcctcttcaa 5700
catattgggg gggtgggtgg ctgcccagct cgccgccccc ggtgccgcta ctgcctttgt 5760
gggtgctggc ctagctggcg ccgccatcgg cagcgttgga ctggggaagg tcctcgtgga 5820
cattcttgca gggtatggcg cgggcgtggc gggagctctt gtagcattca agatcatgag 5880
cggtgaggtc ccctccacgg aggacctggt caatctgctg cccgccatcc tctcgcctgg 5940
agcccttgta gtcggtgtgg tctgcgcagc aatactgcgc cggcacgttg gcccgggcga 6000
gggggcagtg caatggatga accggctaat agccttcgcc tcccggggga accatgtttc 6060
ccccacgcac tacgtgccgg agagcgatgc agccgcccgc gtcactgcca tactcagcag 6120
cctcactgta acccagctcc tgaggcgact gcatcagtgg ataagctcgg agtgtaccac 6180
tccatgctcc ggttcctggc taagggacat ctgggactgg atatgcgagg tgctgagcga 6240
ctttaagacc tggctgaaag ccaagctcat gccacaactg cctgggattc cctttgtgtc 6300
ctgccagcgc gggtataggg gggtctggcg aggagacggc attatgcaca ctcgctgcca 6360
ctgtggagct gagatcactg gacatgtcaa aaacgggacg atgaggatcg tcggtcctag 6420
gacctgcagg aacatgtgga gtgggacgtt ccccattaac gcctacacca cgggcccctg 6480
tactcccctt cctgcgccga actataagtt cgcgctgtgg agggtgtctg cagaggaata 6540
cgtggagata aggcgggtgg gggacttcca ctacgtatcg ggtatgacta ctgacaatct 6600
taaatgcccg tgccagatcc catcgcccga attcttcaca gaattggacg gggtgcgcct 6660
acacaggttt gcgccccctt gcaagccctt gctgcgggag gaggtatcat tcagagtagg 6720
actccacgag tacccggtgg ggtcgcaatt accttgcgag cccgaaccgg acgtagccgt 6780
gttgacgtcc atgctcactg atccctccca tataacagca gaggcggccg ggagaaggtt 6840
ggcgagaggg tcaccccctt ctatggccag ctcctcggct atccagctgt ccgctccatc 6900
tctcaaggca acttgcaccg ccaaccatga ctcccctgac gccgagctca tagaggctaa 6960
cctcctgtgg aggcaggaga tgggcggcaa catcaccagg gttgagtcag agaacaaagt 7020

ggtgattctg gactccttcg atccgcttgt ggcagaggag gatgagcggg aggtctccgt 7080

acctgcagaa attctgcgga agtctcggag attcgcccgg gccctgcccg tctgggcgcg 7140

gccggactac aaccccccgc tagtagagac gtggaaaaag cctgactacg aaccacctgt 7200

ggtccatggc tgcccgctac cacctccacg gtcccctcct gtgcctccgc ctcggaaaaa 7260

gcgtacggtg gtcctcaccg aatcaaccct atctactgcc ttggccgagc ttgccaccaa 7320

aagttttggc agctcctcaa cttccggcat tacgggcgac aatacgacaa catcctctga 7380

gcccgcccct tctggctgcc cccccgactc cgacgttgag tcctattctt ccatgccccc 7440

cctggagggg gagcctgggg atccggatct cagcgacggg tcatggtcga cggtcagtag 7500

tggggccgac acggaagatg tcgtgtgctg ctcaatgtct tattcctgga caggcgcact 7560

cgtcaccccg tgcgctgcgg aagaacaaaa actgcccatc aacgcactga gcaactcgtt 7620

gctacgccat cacaatctgg tgtattccac cacttcacgc agtgcttgcc aaaggcagaa 7680

gaaagtcaca tttgacagac tgcaagttct ggacagccat taccaggacg tgctcaagga 7740

ggtcaaagca gcggcgtcaa aagtgaaggc taacttgcta tccgtagagg aagcttgcag 7800

cctgacgccc ccacattcag ccaaatccaa gtttggctat ggggcaaaag acgtccgttg 7860

ccatgccaga aaggccgtag cccacatcaa ctccgtgtgg aaagaccttc tggaagacag 7920

tgtaacacca attgacacta ccatcatggc caagaacgag gttttctgcg ttcagcctga 7980

gaaggggggt cgtaagccag ctcgtctcat cgtgttcccc gacctgggcg tgcgcgtgtg 8040

cgagaagatg gccctgtacg acgtggttag caagctcccc ctggccgtga tgggaagctc 8100

ctacggattc caatactcac caggacagcg ggttgaattc ctcgtgcaag cgtggaagtc 8160

caagaagacc ccgatggggt tctcgtatga tacccgctgt tttgactcca cagtcactga 8220

gagcgacatc cgtacggagg aggcaattta ccaatgttgt gacctggacc cccaagcccg 8280

cgtggccatc aagtccctca ctgagaggct ttatgttggg ggccctctta ccaattcaag 8340

gggggaaaac tgcggctacc gcaggtgccg cgcgagcggc gtactgacaa ctagctgtgg 8400

taacaccctc acttgctaca tcaaggcccg ggcagcctgt cgagccgcag ggctccagga 8460

ctgcaccatg ctcgtgtgtg gcgacgactt agtcgttatc tgtgaaagtg cgggggtcca 8520

ggaggacgcg gcgagcctga gagccttcac ggaggctatg accaggtact ccgccccccc 8580

cggggacccc ccacaaccag aatacgactt ggagcttata acatcatgct cctccaacgt 8640

gtcagtcgcc cacgacggcg ctggaaagag ggtctactac cttacccgtg accctacaac 8700

ccccctcgcg agagccgcgt gggagacagc aagacacact ccagtcaatt cctggctagg 8760

caacataatc atgtttgccc ccacactgtg ggcgaggatg atactgatga cccatttctt 8820

tagcgtcctc atagccaggg atcagcttga acaggctctt aactgtgaga tctacggagc 8880

ctgctactcc atagaaccac tggatctacc tccaatcatt caaagactcc atggcctcag 8940

cgcattttca ctccacagtt actctccagg tgaaatcaat agggtggccg catgcctcag 9000

aaaacttggg gtcccgccct tgcgagcttg gagacaccgg gcccggagcg tccgcgctag 9060

gcttctgtcc agaggaggca gggctgccat atgtggcaag tacctcttca actgggcagt 9120

aagaacaaag ctcaaactca ctccaatagc ggccgctggc cggctggact tgtccggttg 9180

gttcacggct ggctacagcg ggggagacat ttatcacagc gtgtctcatg cccggccccg 9240

ctggttctgg ttttgcctac tcctgctcgc tgcaggggta ggcatctacc tcctccccaa 9300

ccgatgaagg ttggggtaaa cactccggcc tcttaagcca tttcctgttt tttttttttt 9360

tttttttttt tttttctttt tttttttctt tcctttcctt ctttttttcc tttctttttc 9420

ccttctttaa tggtggctcc atcttagccc tagtcacggc tagctgtgaa aggtccgtga 9480

gccgcttgac tgcagagagt gctgatactg gcctctctgc agatcaagta ctagtagagg 9540

cggtttgcgt attgggcgct cttccgcttc ctcgctcact gactcgctgc gctcggtcgt 9600

tcggctgcgg cgagcggtat cagctcactc aaaggcggta atacggttat ccacagaatc 9660

aggggataac gcaggaaaga acatgtgagc aaaaggccag caaaaggcca ggaaccgtaa 9720

aaaggccgcg ttgctggcgt ttttccatag gctccgcccc cctgacgagc atcacaaaaa 9780

tcgacgctca agtcagaggt ggcgaaaccc gacaggacta taaagatacc aggcgtttcc 9840

ccctggaagc tccctcgtgc gctctcctgt tccgaccctg ccgcttaccg gatacctgtc 9900

cgcctttctc ccttcgggaa gcgtggcgct ttctcatagc tcacgctgta ggtatctcag 9960

ttcggtgtag gtcgttcgct ccaagctggg ctgtgtgcac gaaccccccg ttcagcccga 10020

ccgctgcgcc ttatccggta actatcgtct tgagtccaac ccggtaagac acgacttatc 10080

gccactggca gcagccactg gtaacaggat tagcagagcg aggtatgtag gcggtgctac 10140

agagttcttg aagtggtggc ctaactacgg ctacactaga aggacagtat ttggtatctg 10200

cgctctgctg aagccagtta ccttcggaaa aagagttggt agctcttgat ccggcaaaca 10260

aaccaccgct ggtagcggtg gtttttttgt ttgcaagcag cagattacgc gcagaaaaaa 10320

aggatctcaa gaagatcctt tgatcttttc tacggggtct gacgctcagt ggaacgaaaa 10380

ctcacgttaa gggattttgg tcatgagatt atcaaaaagg atcttcacct agatcctttt 10440

aaattaaaaa tgaagtttta aatcaatcta aagtatatat gagtaaactt ggtctgacag 10500

ttaccaatgc ttaatcagtg aggcacctat ctcagcgatc tgtctatttc gttcatccat 10560

agttgcctga ctccccgtcg tgtagataac tacgatacgg gagggcttac catctggccc 10620

cagtgctgca atgataccgc gagacccacg ctcaccggct ccagatttat cagcaataaa 10680

ccagccagcc ggaagggccg agcgcagaag tggtcctgca actttatccg cctccatcca 10740

gtctattaat tgttgccggg aagctagagt aagtagttcg ccagttaata gtttgcgcaa 10800

cgttgttgcc attgctacag gcatcgtggt gtcacgctcg tcgtttggta tggcttcatt 10860

cagctccggt tcccaacgat caaggcgagt tacatgatcc cccatgttgt gcaaaaaagc 10920

ggttagctcc ttcggtcctc cgatcgttgt cagaagtaag ttggccgcag tgttatcact 10980

catggttatg gcagcactgc ataattctct tactgtcatg ccatccgtaa gatgcttttc 11040

tgtgactggt gagtactcaa ccaagtcatt ctgagaatag tgtatgcggc gaccgagttg 11100

ctcttgcccg gcgtcaatac gggataatac cgcgccacat agcagaactt taaaagtgct 11160

catcattgga aaacgttctt cggggcgaaa actctcaagg atcttaccgc tgttgagatc 11220

cagttcgatg taacccactc gtgcacccaa ctgatcttca gcatctttta ctttcaccag 11280

cgtttctggg tgagcaaaaa caggaaggca aaatgccgca aaaaagggaa taagggcgac 11340

acggaaatgt tgaatactca tactcttcct ttttcaatat tattgaagca tttatcaggg 11400

ttattgtctc atgagcggat acatatttga atgtatttag aaaaataaac aaataggggt 11460

tccgcgcaca tttccccgaa aagtgccacc tgacgtctaa gaaaccatt 11509

<210> 3
<211> 11518
<212> DNA
<213> Artificial Sequence

<220>
<223> Nucleotide sequence of plasmid pSC_1b_NS5B_5`AsiSI_3`RsrII

<400> 3
cctgcaggta atacgactca ctatagccag cccccgattg ggggcgacac tccaccatag 60

atcactcccc tgtgaggaac tactgtcttc acgcagaaag cgtctagcca tggcgttagt 120
atgagtgtcg tgcagcctcc aggacccccc ctcccgggag agccatagtg gtctgcggaa 180
ccggtgagta caccggaatt gccaggacga ccgggtcctt tcttggatca acccgctcaa 240
tgcctggaga tttgggcgtg cccccgcgag actgctagcc gagtagtgtt gggtcgcgaa 300
aggccttgtg gtactgcctg atagggtgct tgcgagtgcc ccgggaggtc tcgtagaccg 360
tgcaccgttt aaacccccgt gctgctggaa gtcgatttca ggcttagggt aaccgtggac 420
ctcgaaaaca gacgcacaaa accaagttca atagaagggg gtacaaacca gtaccaccac 480
gaacaagcac ttctgtttcc ccggtgatgt cgtatagact gcttgcgtgg ttgaaagcga 540
cggatccgtt atccgcttat gtacttcgag aagcccagta ccacctcgga atcttcgatg 600
cgttgcgctc agcactcaac cccagagtgt agcttaggct gatgagtctg gacatccctc 660
accggtgacg gtggtccagg ctgcgttggc ggcctaccta tggctaacgc catgggacgc 720
tagttgtgaa caaggtgtga agagcctatt gagctacata agaatcctcc ggcccctgaa 780
tgcggctaat cccaacctcg gagcaggtgg tcacaaacca gtgattggcc tgtcgtaacg 840
cgcaagtccg tggcggaacc gactactttg ggtgtccgtg tttcctttta ttttattgtg 900
gctgcttatg gtgacaatca cagattgtta tcataaagcg aattggattg gccatccggt 960
gaaagtgaga ctcattatct atctgtttgc tggatccgct ccattgagtg tgtttactct 1020
aagtacaatt tcaacagtta tttcaatcag acaattgtat cataatggcg ggcccagaag 1080
acgccaaaaa cataaagaaa ggcccggcgc cattctatcc tctagaggat ggaaccgctg 1140
gagagcaact gcataaggct atgaagagat acgccctggt tcctggaaca attgctttta 1200
cagatgcaca tatcgaggtg aacatcacgt acgcggaata cttcgaaatg tccgttcggt 1260
tggcagaagc tatgaaacga tatgggctga atacaaatca cagaatcgtc gtatgcagtg 1320
aaaactctct tcaattcttt atgccggtgt tgggcgcgtt atttatcgga gttgcagttg 1380
cgcccgcgaa cgacatttat aatgaacgtg aattgctcaa cagtatgaac atttcgcagc 1440
ctaccgtagt gtttgtttcc aaaaaggggt tgcaaaaaat tttgaacgtg caaaaaaaat 1500
taccaataat ccagaaaatt attatcatgg attctaaaac ggattaccag ggatttcagt 1560
cgatgtacac gttcgtcaca tctcatctac ctcccggttt taatgaatac gattttgtac 1620
cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccagg acctcgtcgg 3600

ctggcaagcg cccccggggg cgcgttcctt gacaccatgc acctgcggca gctcggacct 3660

ttacttggtc acgaggcatg ccgatgtcat tccggtgcgc cggcggggcg acagcagggg 3720

gagcctactc tcccccaggc ccgtctccta cttgaagggc tcttcgggcg gtccactgct 3780

ctgcccctcg gggcacgctg tgggcatctt tcgggctgcc gtgtgcaccc gaggggttgc 3840

gaaggcggtg gactttgtac ccgtcgagtc tatgggaacc actatgcggt ccccggtctt 3900

cacggacaac tcgtcccctc cggccgtacc gcagacattc caggtggccc atctacacgc 3960

ccctactggt agcggcaaga gcactaaggt gccggctgcg tatgcagccc aagggtataa 4020

ggtgcttgtc ctgaacccgt ccgtcgccgc caccctaggt ttcggggcgt atatgtctaa 4080

ggcacatggt atcgacccta acatcagaat cggggtaagg accatcacca cgggtgcccc 4140

catcacgtac tccacctatg gcaagtttct tgccgacggt ggttgctctg gggcgcccta 4200

tgacatcata atatgtgatg agtgccactc aactgactcg accactatcc tgggcatcgg 4260

cacagtcctg gaccaagcgg agacggctgg agcgcgactc gtcgtgctcg ccaccgctac 4320

gcctccggga tcggtcaccg tgccacatcc aaacatcgag gaggtggctc tgtccagcac 4380

tggagaaatc cccttttatg gcaaagccat ccccatcgag accatcaagg gggggaggca 4440

cctcattttc tgccattcca agaagaaatg tgatgagctc gccgcgaagc tgtccggcct 4500

cggactcaat gctgtagcat attaccgggg ccttgatgta tccgtcatac caactagcgg 4560

agacgtcatt gtcgtagcaa cggacgctct aatgacgggc tttaccggcg atttcgactc 4620

agtgatcgac tgcaatacat gtgtcaccca gacagtcgac ttcagcctgg acccgacctt 4680

caccattgag acgacgaccg tgccacaaga cgcggtgtca cgctcgcagc ggcgaggcag 4740

gactggtagg ggcaggatgg gcatttacag gtttgtgact ccaggagaac ggccctcggg 4800

catgttcgat tcctcggttc tgtgcgagtg ctatgacgcg ggctgtgctt ggtacgagct 4860

cacgcccgcc gagacctcag ttaggttgcg ggcttaccta aacacaccag ggttgcccgt 4920

ctgccaggac catctggagt tctgggagag cgtctttaca ggcctcaccc acatagacgc 4980

ccatttcttg tcccagacta agcaggcagg agacaacttc ccctacctgg tagcatacca 5040

ggctacggtg tgcgccaggg ctcaggctcc acctccatcg tgggaccaaa tgtggaagtg 5100

tctcatacgg ctaaagccta cgctgcacgg gccaacgccc ctgctgtata ggctgggagc 5160

cgttcaaaac gaggttacta ccacacaccc cataaccaaa tacatcatgg catgcatgtc 5220

ggctgacctg gaggtcgtca cgagcacctg ggtgctggta ggcggagtcc tagcagctct 5280

ggccgcgtat tgcctgacaa caggcagcgt ggtcattgtg ggcaggatca tcttgtccgg 5340

aaagccggcc atcattcccg acagggaagt cctttaccgg gagttcgatg agatggaaga 5400

gtgcgcctca cacctccctt acatcgaaca gggaatgcag ctcgccgaac aattcaaaca 5460

gaaggcaatc gggttgctgc aaacagccac caagcaagcg gaggctgctg ctcccgtggt 5520

ggaatccaag tggcggaccc tcgaagcctt ctgggcgaag catatgtgga atttcatcag 5580

cgggatacaa tatttagcag gcttgtccac tctgcctggc aaccccgcga tagcatcact 5640

gatggcattc acagcctcta tcaccagccc gctcaccacc caacataccc tcctgtttaa 5700

catcctgggg ggatgggtgg ccgcccaact tgctcctccc agcgctgctt ctgctttcgt 5760

aggcgccggc atcgctggag cggctgttgg cagcataggc cttgggacgg tgcttgtgga 5820

tattttggca ggttatggag caggggtggc aggcgcgctc gtggccttta aggtcatgag 5880

cggcgagatg ccctccaccg aggacctggt taacctactc cctgctatcc tctcccctgg 5940

cgccctagtc gtcggggtcg tgtgcgcagc gatactgcgt cggcacgtgg gcccagggga 6000

gggggctgtg cagtggatga accggctgat agcgttcgct tcgcggggta accacgtctc 6060

ccccacgcac tatgtgcctg agagcgacgc tgcagcacgt gtcactcaga tcctctctag 6120

tcttaccatc actcagctgc tgaagaggct tcaccagtgg atcaacgagg actgctccac 6180

gccatgctcc ggctcgtggc taagagatgt ttgggattgg atatgcacgg tgttgactga 6240

tttcaagacc tggctccagt ccaagctcct gccgcgattg ccgggagtcc ccttcttctc 6300

atgtcaacgt gggtacaagg gagtctggcg gggcgacggc atcatgcaaa ccacctgccc 6360

atgtggagca cagatcaccg gacatgtgaa aaacggttcc atgaggatcg tggggcctag 6420

gacctgtagt aacacgtggc atggaacatt ccccattaac gcgtacacca cgggcccctg 6480

cacgccctcc ccggcgccaa attattctag ggcgctgtgg cgggtggctg ctgaggagta 6540

cgtggaggtt acgcgggtgg gggatttcca ctacgtgacg ggcatgacca ctgacaacgt 6600

aaagtgcccg tgtcaggttc cggcccccga attcttcaca gaagtggatg gggtgcggtt 6660

gcacaggtac gctccagcgt gcaaacccct cctacgggag gaggtcacat tcctggtcgg 6720

gctcaatcaa tacctggttg ggtcacagct cccatgcgag cccgaaccgg acgtagcagt 6780

gctcacttcc atgctcaccg acccctccca cattacggcg gagacggcta agcgtaggct 6840

ggccagggga tctcccccct ccttggccag ctcatcagct agccagctgt ctgcgccttc 6900

cttgaaggca acatgcacta cccgtcatga ctccccggac gctgacctca tcgaggccaa 6960

cctcctgtgg cggcaggaga tgggcgggaa catcacccgc gtggagtcag aaaataaggt 7020

agtaattttg gactctttcg agccgctcca agcggaggag gatgagaggg aagtatccgt 7080

tccggcggag atcctgcgga ggtccaggaa attccctcga gcgatgccca tatgggcacg 7140

cccggattac aaccctccac tgttagagtc ctggaaggac ccggactacg tccctccagt 7200

ggtacacggg tgtccattgc cgcctgccaa ggcccctccg ataccacctc cacggaggaa 7260

gaggacggtt gtcctgtcag aatctaccgt gtcttctgcc ttggcggagc tcgccacaaa 7320

gaccttcggc agctccgaat cgtcggccgt cgacagcggc acggcaacgg cctctcctga 7380

ccagccctcc gacgacggcg acgcgggatc cgacgttgag tcgtactcct ccatgccccc 7440

ccttgagggg gagccggggg atcccgatct cagcgacggg tcttggtcta ccgtaagcga 7500

ggaggctagt gaggacgcga tcgcctgctc gatgtcctac acatggacag gcgccctgat 7560

cacgccatgc gctgcggagg aaaccaagct gcccatcaat gcactgagca actctttgct 7620

ccgtcaccac aacttggtct atgctacaac atctcgcagc gcaagcctgc ggcagaagaa 7680

ggtcaccttt gacagactgc aggtcctgga cgaccactac cgggacgtgc tcaaggagat 7740

gaaggcgaag gcgtccacag ttaaggctaa acttctatcc gtggaggaag cctgtaagct 7800

gacgccccca cattcggcca gatctaaatt tggctatggg gcaaaggacg tccggaacct 7860

atccagcaag gccgttaacc acatccgctc cgtgtggaag gacttgctgg aagacactga 7920

gacaccaatt gacaccacca tcatggcaaa aaatgaggtt ttctgcgtcc aaccagagaa 7980

ggggggccgc aagccagctc gccttatcgt attcccagat ttgggggttc gtgtgtgcga 8040

gaaaatggcc ctttacgatg tggtctccac cctccctcag gccgtgatgg gctcttcata 8100

cggattccaa tactctcctg gacagcgggt cgagttcctg gtgaatgcct ggaaagcgaa 8160

gaaatgccct atgggcttcg catatgacac ccgctgtttt gactcaacgg tcactgagaa 8220

tgacatccgt gttgaggagt caatctacca atgttgtgac ttggcccccg aagccagaca 8280

ggccataagg tcgctcacag agcggcttta catcgggggc cccctgacta attctaaagg 8340

gcagaactgc ggctatcgcc ggtgccgcgc gagcggtgta ctgacgacca gctgcggtaa 8400

taccctcaca tgttacttga aggccgctgc ggcctgtcga gctgcgaagc tccaggactg 8460

cacgatgctc gtatgcggag acgaccttgt cgttatctgt gaaagcgcgg ggacccaaga 8520

ggacgaggcg agcctacggg ccttcacgga ggctatgact agatactctg ccccccctgg 8580

ggacccgccc aaaccagaat acgacttgga gttgataaca tcatgctcct ccaatgtgtc 8640

agtcgcgcac gatgcatctg gcaaaagggt gtactatctc acccgtgacc ccaccacccc 8700

ccttgcgcgg gctgcgtggg agacagctag acacactcca gtcaattcct ggctaggcaa 8760

catcatcatg tatgcgccca ccttgtgggc aaggatgatc ctgatgactc atttcttctc 8820

catccttcta gctcaggaac aacttgaaaa agccctagat tgtcagatct acggggcctg 8880

ttactccatt gagccacttg acctacctca gatcattcaa cgactccatg gccttagcgc 8940

attttcactc catagttact ctccaggtga gatcaatagg gtggcttcat gcctcaggaa 9000

acttggggta ccgcccttgc gagtctggag acatcgggcc agaagtgtcc gcgctaggct 9060

actgtcccag gggggagggc tgccacttgt ggcaagtacc tcttcaactg ggcagtaagg 9120

gaccaagctc aaactcactc caatcccggc tgcgtcccag ttggatttat ccagctggtt 9180

cgttgctggt tacagcgggg gagacatata tcacagcctg tctcgtgccc gaccccgctg 9240

gttcatgtgg tgcctactcc tactttctgt aggggtaggc atctatctac tccccaaccg 9300

atgacggtcc ggctaaacac tccaggccaa taggccatcc tgtttttttc cctttttttt 9360

tttctttttt tttttttttt tttttttttt tttttttctc cttttttttt tcctcttttt 9420

ttccttttct ttcctttggt ggctccatct tagccctagt cacggctagc tgtgaaaggt 9480

ccgtgagccg cttgactgca gagagtgctg atactggcct ctctgcagat caagtactac 9540

tagtagaggc ggtttgcgta ttgggcgctc ttccgcttcc tcgctcactg actcgctgcg 9600

ctcggtcgtt cggctgcggc gagcggtatc agctcactca aaggcggtaa tacggttatc 9660

cacagaatca ggggataacg caggaaagaa catgtgagca aaaggccagc aaaaggccag 9720

gaaccgtaaa aaggccgcgt tgctggcgtt tttccatagg ctccgccccc ctgacgagca 9780

tcacaaaaat cgacgctcaa gtcagaggtg gcgaaacccg acaggactat aaagatacca 9840

ggcgtttccc cctggaagct ccctcgtgcg ctctcctgtt ccgaccctgc cgcttaccgg 9900

atacctgtcc gcctttctcc cttcgggaag cgtggcgctt tctcatagct cacgctgtag 9960

gtatctcagt tcggtgtagg tcgttcgctc caagctgggc tgtgtgcacg aaccccccgt 10020

tcagcccgac cgctgcgcct tatccggtaa ctatcgtctt gagtccaacc cggtaagaca 10080

cgacttatcg ccactggcag cagccactgg taacaggatt agcagagcga ggtatgtagg 10140

cggtgctaca gagttcttga agtggtggcc taactacggc tacactagaa ggacagtatt 10200

tggtatctgc gctctgctga agccagttac cttcggaaaa agagttggta gctcttgatc 10260

cggcaaacaa accaccgctg gtagcggtgg tttttttgtt tgcaagcagc agattacgcg 10320

cagaaaaaaa ggatctcaag aagatccttt gatcttttct acggggtctg acgctcagtg 10380

gaacgaaaac tcacgttaag ggattttggt catgagatta tcaaaaagga tcttcaccta 10440

gatcctttta aattaaaaat gaagttttaa atcaatctaa agtatatatg agtaaacttg 10500

gtctgacagt taccaatgct taatcagtga ggcacctatc tcagcgatct gtctatttcg 10560

ttcatccata gttgcctgac tccccgtcgt gtagataact acgatacggg agggcttacc 10620

atctggcccc agtgctgcaa tgataccgcg agacccacgc tcaccggctc cagatttatc 10680

agcaataaac cagccagccg gaagggccga gcgcagaagt ggtcctgcaa ctttatccgc 10740

ctccatccag tctattaatt gttgccggga agctagagta agtagttcgc cagttaatag 10800

tttgcgcaac gttgttgcca ttgctacagg catcgtggtg tcacgctcgt cgtttggtat 10860

ggcttcattc agctccggtt cccaacgatc aaggcgagtt acatgatccc ccatgttgtg 10920

caaaaaagcg gttagctcct tcggtcctcc gatcgttgtc agaagtaagt tggccgcagt 10980

gttatcactc atggttatgg cagcactgca taattctctt actgtcatgc catccgtaag 11040

atgcttttct gtgactggtg agtactcaac caagtcattc tgagaatagt gtatgcggcg 11100

accgagttgc tcttgcccgg cgtcaatacg ggataatacc gcgccacata gcagaacttt 11160

aaaagtgctc atcattggaa aacgttcttc ggggcgaaaa ctctcaagga tcttaccgct 11220

gttgagatcc agttcgatgt aacccactcg tgcacccaac tgatcttcag catcttttac 11280

tttcaccagc gtttctgggt gagcaaaaac aggaaggcaa aatgccgcaa aaaagggaat 11340

aagggcgaca cggaaatgtt gaatactcat actcttcctt tttcaatatt attgaagcat 11400

ttatcagggt tattgtctca tgagcggata catatttgaa tgtatttaga aaaataaaca 11460

aataggggtt ccgcgcacat ttccccgaaa agtgccacct gacgtctaag aaaccatt 11518

<210> 4
<211> 11518
<212> DNA
<213> Artificial Sequence

<220>
<223> Nucleotide Sequence of plasmid pSC_1b_NS5B_5`AsiSI_3`SacII

<400> 4
cctgcaggta atacgactca ctatagccag cccccgattg ggggcgacac tccaccatag 60

atcactcccc tgtgaggaac tactgtcttc acgcagaaag cgtctagcca tggcgttagt 120

atgagtgtcg tgcagcctcc aggacccccc ctcccgggag agccatagtg gtctgcggaa 180

ccggtgagta caccggaatt gccaggacga ccgggtcctt tcttggatca acccgctcaa 240

tgcctggaga tttgggcgtg cccccgcgag actgctagcc gagtagtgtt gggtcgcgaa 300

aggccttgtg gtactgcctg atagggtgct tgcgagtgcc ccgggaggtc tcgtagaccg 360

tgcaccgttt aaacccccgt gctgctggaa gtcgatttca ggcttagggt aaccgtggac 420

ctcgaaaaca gacgcacaaa accaagttca atagaagggg gtacaaacca gtaccaccac 480

gaacaagcac ttctgtttcc ccggtgatgt cgtatagact gcttgcgtgg ttgaaagcga 540

cggatccgtt atccgcttat gtacttcgag aagcccagta ccacctcgga atcttcgatg 600

cgttgcgctc agcactcaac cccagagtgt agcttaggct gatgagtctg gacatccctc 660

accggtgacg gtggtccagg ctgcgttggc ggcctaccta tggctaacgc catgggacgc 720

tagttgtgaa caaggtgtga agagcctatt gagctacata agaatcctcc ggcccctgaa 780

tgcggctaat cccaacctcg gagcaggtgg tcacaaacca gtgattggcc tgtcgtaacg 840

cgcaagtccg tggcggaacc gactactttg ggtgtccgtg tttcctttta ttttattgtg 900

gctgcttatg gtgacaatca cagattgtta tcataaagcg aattggattg gccatccggt 960

gaaagtgaga ctcattatct atctgtttgc tggatccgct ccattgagtg tgtttactct 1020

aagtacaatt tcaacagtta tttcaatcag acaattgtat cataatggcg ggcccagaag 1080

acgccaaaaa cataaagaaa ggcccggcgc cattctatcc tctagaggat ggaaccgctg 1140

gagagcaact gcataaggct atgaagagat acgccctggt tcctggaaca attgctttta 1200

cagatgcaca tatcgaggtg aacatcacgt acgcggaata cttcgaaatg tccgttcggt 1260

tggcagaagc tatgaaacga tatgggctga atacaaatca cagaatcgtc gtatgcagtg 1320

aaaactctct tcaattcttt atgccggtgt tgggcgcgtt atttatcgga gttgcagttg 1380

cgcccgcgaa cgacatttat aatgaacgtg aattgctcaa cagtatgaac atttcgcagc 1440

ctaccgtagt gtttgtttcc aaaaaggggt tgcaaaaaat tttgaacgtg caaaaaaaat 1500

taccaataat ccagaaaatt attatcatgg attctaaaac ggattaccag ggatttcagt 1560

cgatgtacac gttcgtcaca tctcatctac ctcccggttt taatgaatac gattttgtac 1620

cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccagg acctcgtcgg 3600

ctggcaagcg ccccccgggg cgcgttcctt gacaccatgc acctgcggca gctcggacct 3660

ttacttggtc acgaggcatg ccgatgtcat tccggtgcgc cggcggggcg acagcagggg 3720

gagcctactc tcccccaggc ccgtctccta cttgaagggc tcttcgggcg gtccactgct 3780

ctgcccctcg gggcacgctg tgggcatctt tcgggctgcc gtgtgcaccc gaggggttgc 3840

gaaggcggtg gactttgtac ccgtcgagtc tatgggaacc actatgcggt ccccggtctt 3900

cacggacaac tcgtcccctc cggccgtacc gcagacattc caggtggccc atctacacgc 3960

ccctactggt agcggcaaga gcactaaggt gccggctgcg tatgcagccc aagggtataa 4020

ggtgcttgtc ctgaacccgt ccgtcgccgc caccctaggt ttcggggcgt atatgtctaa 4080

ggcacatggt atcgacccta acatcagaat cggggtaagg accatcacca cgggtgcccc 4140

catcacgtac tccacctatg gcaagtttct tgccgacggt ggttgctctg gggggcgcta 4200

tgacatcata atatgtgatg agtgccactc aactgactcg accactatcc tgggcatcgg 4260

cacagtcctg gaccaagcgg agacggctgg agcgcgactc gtcgtgctcg ccaccgctac 4320

gcctccggga tcggtcaccg tgccacatcc aaacatcgag gaggtggctc tgtccagcac 4380

tggagaaatc cccttttatg gcaaagccat ccccatcgag accatcaagg gggggaggca 4440

cctcattttc tgccattcca agaagaaatg tgatgagctc gccgcgaagc tgtccggcct 4500
cggactcaat gctgtagcat attaccgggg ccttgatgta tccgtcatac caactagcgg 4560
agacgtcatt gtcgtagcaa cggacgctct aatgacgggc tttaccggcg atttcgactc 4620
agtgatcgac tgcaatacat gtgtcaccca gacagtcgac ttcagcctgg acccgacctt 4680
caccattgag acgacgaccg tgccacaaga cgcggtgtca cgctcgcagc ggcgaggcag 4740
gactggtagg ggcaggatgg gcatttacag gtttgtgact ccaggagaac ggccctcggg 4800
catgttcgat tcctcggttc tgtgcgagtg ctatgacgcg ggctgtgctt ggtacgagct 4860
cacgcccgcc gagacctcag ttaggttgcg ggcttaccta aacacaccag ggttgcccgt 4920
ctgccaggac catctggagt tctgggagag cgtctttaca ggcctcaccc acatagacgc 4980
ccatttcttg tcccagacta agcaggcagg agacaacttc ccctacctgg tagcatacca 5040
ggctacggtg tgcgccaggg ctcaggctcc acctccatcg tgggaccaaa tgtggaagtg 5100
tctcatacgg ctaaagccta cgctgcacgg gccaacgccc ctgctgtata ggctgggagc 5160
cgttcaaaac gaggttacta ccacacaccc cataaccaaa tacatcatgg catgcatgtc 5220
ggctgacctg gaggtcgtca cgagcacctg ggtgctggta ggcggagtcc tagcagctct 5280
ggccgcgtat tgcctgacaa caggcagcgt ggtcattgtg ggcaggatca tcttgtccgg 5340
aaagccggcc atcattcccg acagggaagt cctttaccgg gagttcgatg agatggaaga 5400
gtgcgcctca cacctccctt acatcgaaca gggaatgcag ctcgccgaac aattcaaaca 5460
gaaggcaatc gggttgctgc aaacagccac caagcaagcg gaggctgctg ctcccgtggt 5520
ggaatccaag tggcggaccc tcgaagcctt ctgggcgaag catatgtgga atttcatcag 5580
cgggatacaa tatttagcag gcttgtccac tctgcctggc aaccccgcga tagcatcact 5640
gatggcattc acagcctcta tcaccagccc gctcaccacc caacataccc tcctgtttaa 5700
catcctgggg ggatgggtgg ccgcccaact tgctcctccc agcgctgctt ctgctttcgt 5760
aggcgccggc atcgctggag cggctgttgg cagcataggc cttgggacgg tgcttgtgga 5820
tattttggca ggttatggag caggggtggc aggcgcgctc gtggccttta aggtcatgag 5880
cggcgagatg ccctccaccg aggacctggt taacctactc cctgctatcc tctcccctgg 5940
cgccctagtc gtcggggtcg tgtgcgcagc gatactgcgt cggcacgtgg gcccagggga 6000
gggggctgtg cagtggatga accggctgat agcgttcgct tcgcggggta accacgtctc 6060

ccccacgcac tatgtgcctg agagcgacgc tgcagcacgt gtcactcaga tcctctctag 6120

tcttaccatc actcagctgc tgaagaggct tcaccagtgg atcaacgagg actgctccac 6180

gccatgctcc ggctcgtggc taagagatgt ttgggattgg atatgcacgg tgttgactga 6240

tttcaagacc tggctccagt ccaagctcct gccgcgattg ccgggagtcc ccttcttctc 6300

atgtcaacgt gggtacaagg gagtctggcg gggcgacggc atcatgcaaa ccacctgccc 6360

atgtggagca cagatcaccg gacatgtgaa aaacggttcc atgaggatcg tggggcctag 6420

gacctgtagt aacacgtggc atggaacatt ccccattaac gcgtacacca cgggcccctg 6480

cacgccctcc ccggcgccaa attattctag ggcgctgtgg cgggtggctg ctgaggagta 6540

cgtggaggtt acgcgggtgg gggatttcca ctacgtgacg ggcatgacca ctgacaacgt 6600

aaagtgcccg tgtcaggttc cggcccccga attcttcaca gaagtggatg gggtgcggtt 6660

gcacaggtac gctccagcgt gcaaacccct cctacgggag gaggtcacat tcctggtcgg 6720

gctcaatcaa tacctggttg ggtcacagct cccatgcgag cccgaaccgg acgtagcagt 6780

gctcacttcc atgctcaccg acccctccca cattacggcg gagacggcta agcgtaggct 6840

ggccagggga tctcccccct ccttggccag ctcatcagct agccagctgt ctgcgccttc 6900

cttgaaggca acatgcacta cccgtcatga ctccccggac gctgacctca tcgaggccaa 6960

cctcctgtgg cggcaggaga tgggcgggaa catcacccgc gtggagtcag aaaataaggt 7020

agtaattttg gactctttcg agccgctcca agcggaggag gatgagaggg aagtatccgt 7080

tccggcggag atcctgcgga ggtccaggaa attccctcga gcgatgccca tatgggcacg 7140

cccggattac aaccctccac tgttagagtc ctggaaggac ccggactacg tccctccagt 7200

ggtacacggg tgtccattgc cgcctgccaa ggcccctccg ataccacctc cacggaggaa 7260

gaggacggtt gtcctgtcag aatctaccgt gtcttctgcc ttggcggagc tcgccacaaa 7320

gaccttcggc agctccgaat cgtcggccgt cgacagcggc acggcaacgg cctctcctga 7380

ccagccctcc gacgacggcg acgcgggatc cgacgttgag tcgtactcct ccatgccccc 7440

ccttgagggg gagccggggg atcccgatct cagcgacggg tcttggtcta ccgtaagcga 7500

ggaggctagt gaggacgcga tcgcctgctc gatgtcctac acatggacag gcgccctgat 7560

cacgccatgc gctgcggagg aaaccaagct gcccatcaat gcactgagca actctttgct 7620

ccgtcaccac aacttggtct atgctacaac atctcgcagc gcaagcctgc ggcagaagaa 7680

ggtcaccttt gacagactgc aggtcctgga cgaccactac cgggacgtgc tcaaggagat 7740

gaaggcgaag gcgtccacag ttaaggctaa acttctatcc gtggaggaag cctgtaagct 7800

gacgccccca cattcggcca gatctaaatt tggctatggg gcaaaggacg tccggaacct 7860

atccagcaag gccgttaacc acatccgctc cgtgtggaag gacttgctgg aagacactga 7920

gacaccaatt gacaccacca tcatggcaaa aaatgaggtt ttctgcgtcc aaccagagaa 7980

ggggggccgc aagccagctc gccttatcgt attcccagat ttgggggttc gtgtgtgcga 8040

gaaaatggcc ctttacgatg tggtctccac cctccctcag gccgtgatgg gctcttcata 8100

cggattccaa tactctcctg gacagcgggt cgagttcctg gtgaatgcct ggaaagcgaa 8160

gaaatgccct atgggcttcg catatgacac ccgctgtttt gactcaacgg tcactgagaa 8220

tgacatccgt gttgaggagt caatctacca atgttgtgac ttggcccccg aagccagaca 8280

ggccataagg tcgctcacag agcggcttta catcgggggc cccctgacta attctaaagg 8340

gcagaactgc ggctatcgcc ggtgccgcgc gagcggtgta ctgacgacca gctgcggtaa 8400

taccctcaca tgttacttga aggccgctgc ggcctgtcga gctgcgaagc tccaggactg 8460

cacgatgctc gtatgcggag acgaccttgt cgttatctgt gaaagcgcgg ggacccaaga 8520

ggacgaggcg agcctacggg ccttcacgga ggctatgact agatactctg ccccccctgg 8580

ggacccgccc aaaccagaat acgacttgga gttgataaca tcatgctcct ccaatgtgtc 8640

agtcgcgcac gatgcatctg gcaaaagggt gtactatctc acccgtgacc ccaccacccc 8700

ccttgcgcgg gctgcgtggg agacagctag acacactcca gtcaattcct ggctaggcaa 8760

catcatcatg tatgcgccca ccttgtgggc aaggatgatc ctgatgactc atttcttctc 8820

catccttcta gctcaggaac aacttgaaaa agccctagat tgtcagatct acggggcctg 8880

ttactccatt gagccacttg acctacctca gatcattcaa cgactccatg gccttagcgc 8940

attttcactc catagttact ctccaggtga gatcaatagg gtggcttcat gcctcaggaa 9000

acttggggta ccgcccttgc gagtctggag acatcgggcc agaagtgtcc gcgctaggct 9060

actgtcccag ggggggaggg ctgccacttg tggcaagtac ctcttcaact gggcagtaag 9120

gaccaagctc aaactcactc caatcccggc tgcgtcccag ttggatttat ccagctggtt 9180

cgttgctggt tacagcgggg gagacatata tcacagcctg tctcgtgccc gaccccgctg 9240

gttcatgtgg tgcctactcc tactttctgt aggggtaggc atctatctac tccccaaccg 9300

atgaccgcgg agctaaacac tccaggccaa taggccatcc tgtttttttc cctttttttt 9360

tttctttttt tttttttttt tttttttttt ttttttttct cctttttttt tcctcttttt 9420

ttccttttct ttcctttggt ggctccatct tagccctagt cacggctagc tgtgaaaggt 9480

ccgtgagccg cttgactgca gagagtgctg atactggcct ctctgcagat caagtactac 9540

tagtagaggc ggtttgcgta ttgggcgctc ttccgcttcc tcgctcactg actcgctgcg 9600

ctcggtcgtt cggctgcggc gagcggtatc agctcactca aaggcggtaa tacggttatc 9660

cacagaatca ggggataacg caggaaagaa catgtgagca aaaggccagc aaaaggccag 9720

gaaccgtaaa aaggccgcgt tgctggcgtt tttccatagg ctccgccccc ctgacgagca 9780

tcacaaaaat cgacgctcaa gtcagaggtg gcgaaacccg acaggactat aaagatacca 9840

ggcgtttccc cctggaagct ccctcgtgcg ctctcctgtt ccgaccctgc cgcttaccgg 9900

atacctgtcc gcctttctcc cttcgggaag cgtggcgctt tctcatagct cacgctgtag 9960

gtatctcagt tcggtgtagg tcgttcgctc caagctgggc tgtgtgcacg aaccccccgt 10020

tcagcccgac cgctgcgcct tatccggtaa ctatcgtctt gagtccaacc cggtaagaca 10080

cgacttatcg ccactggcag cagccactgg taacaggatt agcagagcga ggtatgtagg 10140

cggtgctaca gagttcttga agtggtggcc taactacggc tacactagaa ggacagtatt 10200

tggtatctgc gctctgctga agccagttac cttcggaaaa agagttggta gctcttgatc 10260

cggcaaacaa accaccgctg gtagcggtgg tttttttgtt tgcaagcagc agattacgcg 10320

cagaaaaaaa ggatctcaag aagatccttt gatcttttct acggggtctg acgctcagtg 10380

gaacgaaaac tcacgttaag ggattttggt catgagatta tcaaaaagga tcttcaccta 10440

gatcctttta aattaaaaat gaagttttaa atcaatctaa agtatatatg agtaaacttg 10500

gtctgacagt taccaatgct taatcagtga ggcacctatc tcagcgatct gtctatttcg 10560

ttcatccata gttgcctgac tccccgtcgt gtagataact acgatacggg agggcttacc 10620

atctggcccc agtgctgcaa tgataccgcg agacccacgc tcaccggctc cagatttatc 10680

agcaataaac cagccagccg gaagggccga gcgcagaagt ggtcctgcaa ctttatccgc 10740

ctccatccag tctattaatt gttgccggga agctagagta agtagttcgc cagttaatag 10800

tttgcgcaac gttgttgcca ttgctacagg catcgtggtg tcacgctcgt cgtttggtat 10860

ggcttcattc agctccggtt cccaacgatc aaggcgagtt acatgatccc ccatgttgtg 10920

caaaaaagcg gttagctcct tcggtcctcc gatcgttgtc agaagtaagt tggccgcagt 10980

gttatcactc atggttatgg cagcactgca taattctctt actgtcatgc catccgtaag 11040

atgcttttct gtgactggtg agtactcaac caagtcattc tgagaatagt gtatgcggcg 11100

accgagttgc tcttgcccgg cgtcaatacg ggataatacc gcgccacata gcagaacttt 11160

aaaagtgctc atcattggaa aacgttcttc ggggcgaaaa ctctcaagga tcttaccgct 11220

gttgagatcc agttcgatgt aacccactcg tgcacccaac tgatcttcag catcttttac 11280

tttcaccagc gtttctgggt gagcaaaaac aggaaggcaa aatgccgcaa aaaagggaat 11340

aagggcgaca cggaaatgtt gaatactcat actcttcctt tttcaatatt attgaagcat 11400

ttatcagggt tattgtctca tgagcggata catatttgaa tgtatttaga aaaataaaca 11460

aataggggtt ccgcgcacat ttccccgaaa agtgccacct gacgtctaag aaaccatt 11518

<210> 5
<211> 10473
<212> DNA
<213> Artificial Sequence

<220>
<223> Nucleotide sequence of plasmid pSC_1b_delNS5B_5`AsiSI_3`SacII

<400> 5
cctgcaggta atacgactca ctatagccag cccccgattg ggggcgacac tccaccatag 60

atcactcccc tgtgaggaac tactgtcttc acgcagaaag cgtctagcca tggcgttagt 120

atgagtgtcg tgcagcctcc aggacccccc ctcccgggag agccatagtg gtctgcggaa 180

ccggtgagta caccggaatt gccaggacga ccgggtcctt tcttggatca acccgctcaa 240

tgcctggaga tttgggcgtg cccccgcgag actgctagcc gagtagtgtt gggtcgcgaa 300

aggccttgtg gtactgcctg atagggtgct tgcgagtgcc ccgggaggtc tcgtagaccg 360

tgcaccgttt aaacccccgt gctgctggaa gtcgatttca ggcttagggt aaccgtggac 420

ctcgaaaaca gacgcacaaa accaagttca atagaagggg gtacaaacca gtaccaccac 480

gaacaagcac ttctgtttcc ccggtgatgt cgtatagact gcttgcgtgg ttgaaagcga 540

cggatccgtt atccgcttat gtacttcgag aagcccagta ccacctcgga atcttcgatg 600

cgttgcgctc agcactcaac cccagagtgt agcttaggct gatgagtctg gacatccctc 660

accggtgacg gtggtccagg ctgcgttggc ggcctaccta tggctaacgc catgggacgc 720

tagttgtgaa caaggtgtga agagcctatt gagctacata agaatcctcc ggcccctgaa 780

tgcggctaat cccaacctcg gagcaggtgg tcacaaacca gtgattggcc tgtcgtaacg 840

cgcaagtccg tggcggaacc gactactttg ggtgtccgtg tttcctttta ttttattgtg 900

gctgcttatg gtgacaatca cagattgtta tcataaagcg aattggattg gccatccggt 960

gaaagtgaga ctcattatct atctgtttgc tggatccgct ccattgagtg tgtttactct 1020

aagtacaatt tcaacagtta tttcaatcag acaattgtat cataatggcg ggcccagaag 1080

acgccaaaaa cataaagaaa ggcccggcgc cattctatcc tctagaggat ggaaccgctg 1140

gagagcaact gcataaggct atgaagagat acgccctggt tcctggaaca attgctttta 1200

cagatgcaca tatcgaggtg aacatcacgt acgcggaata cttcgaaatg tccgttcggt 1260

tggcagaagc tatgaaacga tatgggctga atacaaatca cagaatcgtc gtatgcagtg 1320

aaaactctct tcaattcttt atgccggtgt tgggcgcgtt atttatcgga gttgcagttg 1380

cgcccgcgaa cgacatttat aatgaacgtg aattgctcaa cagtatgaac atttcgcagc 1440

ctaccgtagt gtttgtttcc aaaaaggggt tgcaaaaaat tttgaacgtg caaaaaaaat 1500

taccaataat ccagaaaatt attatcatgg attctaaaac ggattaccag ggatttcagt 1560

cgatgtacac gttcgtcaca tctcatctac ctcccggttt taatgaatac gattttgtac 1620

cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccagg acctcgtcgg 3600

ctggcaagcg ccccccgggg cgcgttcctt gacaccatgc acctgcggca gctcggacct 3660

ttacttggtc acgaggcatg ccgatgtcat tccggtgcgc cggcggggcg acagcagggg 3720

gagcctactc tcccccaggc ccgtctccta cttgaagggc tcttcgggcg gtccactgct 3780

ctgcccctcg gggcacgctg tgggcatctt tcgggctgcc gtgtgcaccc gaggggttgc 3840

gaaggcggtg gactttgtac ccgtcgagtc tatgggaacc actatgcggt ccccggtctt 3900

cacggacaac tcgtcccctc cggccgtacc gcagacattc caggtggccc atctacacgc 3960

ccctactggt agcggcaaga gcactaaggt gccggctgcg tatgcagccc aagggtataa 4020

ggtgcttgtc ctgaacccgt ccgtcgccgc caccctaggt ttcggggcgt atatgtctaa 4080

ggcacatggt atcgacccta acatcagaat cggggtaagg accatcacca cgggtgcccc 4140

catcacgtac tccacctatg gcaagtttct tgccgacggt ggttgctctg gggcgcccta 4200

tgacatcata atatgtgatg agtgccactc aactgactcg accactatcc tgggcatcgg 4260

cacagtcctg gaccaagcgg agacggctgg agcgcgactc gtcgtgctcg ccaccgctac 4320

gcctccggga tcggtcaccg tgccacatcc aaacatcgag gaggtggctc tgtccagcac 4380

tggagaaatc cccttttatg gcaaagccat ccccatcgag accatcaagg gggggaggca 4440

cctcattttc tgccattcca agaagaaatg tgatgagctc gccgcgaagc tgtccggcct 4500

cggactcaat gctgtagcat attaccgggg ccttgatgta tccgtcatac caactagcgg 4560

agacgtcatt gtcgtagcaa cggacgctct aatgacgggc tttaccggcg atttcgactc 4620

agtgatcgac tgcaatacat gtgtcaccca gacagtcgac ttcagcctgg acccgacctt 4680

caccattgag acgacgaccg tgccacaaga cgcggtgtca cgctcgcagc ggcgaggcag 4740

gactggtagg ggcaggatgg gcatttacag gtttgtgact ccaggagaac ggccctcggg 4800

catgttcgat tcctcggttc tgtgcgagtg ctatgacgcg ggctgtgctt ggtacgagct 4860

cacgcccgcc gagacctcag ttaggttgcg ggcttaccta aacacaccag ggttgcccgt 4920

ctgccaggac catctggagt tctgggagag cgtctttaca ggcctcaccc acatagacgc 4980

ccatttcttg tcccagacta agcaggcagg agacaacttc ccctacctgg tagcatacca 5040

ggctacggtg tgcgccaggg ctcaggctcc acctccatcg tgggaccaaa tgtggaagtg 5100

tctcatacgg ctaaagccta cgctgcacgg gccaacgccc ctgctgtata ggctgggagc 5160

cgttcaaaac gaggttacta ccacacaccc cataaccaaa tacatcatgg catgcatgtc 5220

ggctgacctg gaggtcgtca cgagcacctg ggtgctggta ggcggagtcc tagcagctct 5280

ggccgcgtat tgcctgacaa caggcagcgt ggtcattgtg ggcaggatca tcttgtccgg 5340

aaagccggcc atcattcccg acagggaagt cctttaccgg gagttcgatg agatggaaga 5400

gtgcgcctca cacctccctt acatcgaaca gggaatgcag ctcgccgaac aattcaaaca 5460

gaaggcaatc gggttgctgc aaacagccac caagcaagcg gaggctgctg ctcccgtggt 5520

ggaatccaag tggcggaccc tcgaagcctt ctgggcgaag catatgtgga atttcatcag 5580

cgggatacaa tatttagcag gcttgtccac tctgcctggc aaccccgcga tagcatcact 5640
gatggcattc acagcctcta tcaccagccc gctcaccacc caacataccc tcctgtttaa 5700
catcctgggg ggatgggtgg ccgcccaact tgctcctccc agcgctgctt ctgctttcgt 5760
aggcgccggc atcgctggag cggctgttgg cagcataggc cttgggacgg tgcttgtgga 5820
tattttggca ggttatggag caggggtggc aggcgcgctc gtggccttta aggtcatgag 5880
cggcgagatg ccctccaccg aggacctggt taacctactc cctgctatcc tctcccctgg 5940
cgccctagtc gtcggggtcg tgtgcgcagc gatactgcgt cggcacgtgg gcccagggga 6000
gggggctgtg cagtggatga accggctgat agcgttcgct tcgcggggta accacgtctc 6060
ccccacgcac tatgtgcctg agagcgacgc tgcagcacgt gtcactcaga tcctctctag 6120
tcttaccatc actcagctgc tgaagaggct tcaccagtgg atcaacgagg actgctccac 6180
gccatgctcc ggctcgtggc taagagatgt ttgggattgg atatgcacgg tgttgactga 6240
tttcaagacc tggctccagt ccaagctcct gccgcgattg ccgggagtcc ccttcttctc 6300
atgtcaacgt gggtacaagg gagtctggcg gggcgacggc atcatgcaaa ccacctgccc 6360
atgtggagca cagatcaccg gacatgtgaa aaacggttcc atgaggatcg tggggcctag 6420
gacctgtagt aacacgtggc atggaacatt ccccattaac gcgtacacca cgggcccctg 6480
cacgccctcc ccggcgccaa attattctag ggcgctgtgg cgggtggctg ctgaggagta 6540
cgtggaggtt acgcgggtgg gggatttcca ctacgtgacg ggcatgacca ctgacaacgt 6600
aaagtgcccg tgtcaggttc cggcccccga attcttcaca gaagtggatg gggtgcggtt 6660
gcacaggtac gctccagcgt gcaaacccct cctacgggag gaggtcacat tcctggtcgg 6720
gctcaatcaa tacctggttg ggtcacagct cccatgcgag cccgaaccgg acgtagcagt 6780
gctcacttcc atgctcaccg acccctccca cattacggcg gagacggcta agcgtaggct 6840
ggccagggga tctcccccct ccttggccag ctcatcagct agccagctgt ctgcgccttc 6900
cttgaaggca acatgcacta cccgtcatga ctccccggac gctgacctca tcgaggccaa 6960
cctcctgtgg cggcaggaga tgggcgggaa catcacccgc gtggagtcag aaaataaggt 7020
agtaattttg gactctttcg agccgctcca agcggaggag gatgagaggg aagtatccgt 7080
tccggcggag atcctgcgga ggtccaggaa attccctcga gcgatgccca tatgggcacg 7140
cccggattac aaccctccac tgttagagtc ctggaaggac ccggactacg tccctccagt 7200

ggtacacggg tgtccattgc cgcctgccaa ggcccctccg ataccacctc cacggaggaa 7260

gaggacggtt gtcctgtcag aatctaccgt gtcttctgcc ttggcggagc tcgccacaaa 7320

gaccttcggc agctccgaat cgtcggccgt cgacagcggc acggcaacgg cctctcctga 7380

ccagccctcc gacgacggcg acgcgggatc cgacgttgag tcgtactcct ccatgccccc 7440

ccttgagggg gagccggggg atcccgatct cagcgacggg tcttggtcta ccgtaagcga 7500

ggaggctagt gaggacgcga tcgcctgctc gatgtcctac acatggacag gcgccctgat 7560

cacgccatgc gctgcggagg aaaccaagct gcccatcaat gcactgagca actctttgct 7620

ccgtcaccac aacttggtct atgctacaac atctcgcagc gcaagcctgc ggcagaagaa 7680

ggtcaccttt gacagactgc aggtcctgga cgaccactac cgggacgtgc tcaaggagat 7740

gaaggcgaag gcgtccacag ttaaggctaa acttctatcc gtggaggaag cctgtaagct 7800

gacgccccca cattcggcca gatctacggg gcctgttact ccattgagcc acttgaccta 7860

cctcagatca ttcaacgact ccatggcctt agcgcatttt cactccatag ttactctcca 7920

ggtgagatca atagggtggc ttcatgcctc aggaaacttg ggtaccgccc cttgcgagtc 7980

tggagacatc gggccagaag tgtccgcgct aggctactgt cccagggggg gagggctgcc 8040

acttgtggca agtacctctt caactgggca gtaaggacca agctcaaact cactccaatc 8100

ccggctgcgt cccagttgga tttatccagc tggttcgttg ctggttacag cgggggagac 8160

atatatcaca gcctgtctcg tgcccgaccc cgctggttca tgtggtgcct actcctactt 8220

tctgtagggg taggcatcta tctactcccc aaccgatgac cgcggagcta aacactccag 8280

gccaataggc catcctgttt ttttcccttt ttttttttct tttttttttt tttttttttt 8340

tttttttttt tttctccttt ttttttcctc tttttttcct tttctttcct ttggtggctc 8400

catcttagcc ctagtcacgg ctagctgtga aaggtccgtg agccgcttga ctgcagagag 8460

tgctgatact ggcctctctg cagatcaagt actactagta gaggcggttt gcgtattggg 8520

cgctcttccg cttcctcgct cactgactcg ctgcgctcgg tcgttcggct gcggcgagcg 8580

gtatcagctc actcaaaggc ggtaatacgg ttatccacag aatcagggga taacgcagga 8640

aagaacatgt gagcaaaagg ccagcaaaag gccaggaacc gtaaaaaggc cgcgttgctg 8700

gcgtttttcc ataggctccg cccccctgac gagcatcaca aaaatcgacg ctcaagtcag 8760

aggtggcgaa acccgacagg actataaaga taccaggcgt ttccccctgg aagctccctc 8820

gtgcgctctc ctgttccgac cctgccgctt accggatacc tgtccgcctt tctcccttcg 8880

ggaagcgtgg cgctttctca tagctcacgc tgtaggtatc tcagttcggt gtaggtcgtt 8940

cgctccaagc tgggctgtgt gcacgaaccc cccgttcagc ccgaccgctg cgccttatcc 9000

ggtaactatc gtcttgagtc caacccggta agacacgact tatcgccact ggcagcagcc 9060

actggtaaca ggattagcag agcgaggtat gtaggcggtg ctacagagtt cttgaagtgg 9120

tggcctaact acggctacac tagaaggaca gtatttggta tctgcgctct gctgaagcca 9180

gttaccttcg gaaaaagagt tggtagctct tgatccggca aacaaaccac cgctggtagc 9240

ggtggttttt ttgtttgcaa gcagcagatt acgcgcagaa aaaaaggatc tcaagaagat 9300

cctttgatct tttctacggg gtctgacgct cagtggaacg aaaactcacg ttaagggatt 9360

ttggtcatga gattatcaaa aaggatcttc acctagatcc ttttaaatta aaaatgaagt 9420

tttaaatcaa tctaaagtat atatgagtaa acttggtctg acagttacca atgcttaatc 9480

agtgaggcac ctatctcagc gatctgtcta tttcgttcat ccatagttgc ctgactcccc 9540

gtcgtgtaga taactacgat acgggagggc ttaccatctg gccccagtgc tgcaatgata 9600

ccgcgagacc cacgctcacc ggctccagat ttatcagcaa taaaccagcc agccggaagg 9660

gccgagcgca gaagtggtcc tgcaacttta tccgcctcca tccagtctat taattgttgc 9720

cgggaagcta gagtaagtag ttcgccagtt aatagtttgc gcaacgttgt tgccattgct 9780

acaggcatcg tggtgtcacg ctcgtcgttt ggtatggctt cattcagctc cggttcccaa 9840

cgatcaaggc gagttacatg atcccccatg ttgtgcaaaa aagcggttag ctccttcggt 9900

cctccgatcg ttgtcagaag taagttggcc gcagtgttat cactcatggt tatggcagca 9960

ctgcataatt ctcttactgt catgccatcc gtaagatgct tttctgtgac tggtgagtac 10020

tcaaccaagt cattctgaga atagtgtatg cggcgaccga gttgctcttg cccggcgtca 10080

atacgggata ataccgcgcc acatagcaga actttaaaag tgctcatcat tggaaaacgt 10140

tcttcggggc gaaaactctc aaggatctta ccgctgttga gatccagttc gatgtaaccc 10200

actcgtgcac ccaactgatc ttcagcatct tttactttca ccagcgtttc tgggtgagca 10260

aaaacaggaa ggcaaaatgc cgcaaaaaag ggaataaggg cgacacggaa atgttgaata 10320

ctcatactct tcctttttca atattattga agcatttatc agggttattg tctcatgagc 10380

ggatacatat ttgaatgtat ttagaaaaat aaacaaatag gggttccgcg cacatttccc 10440

cgaaaagtgc cacctgacgt ctaagaaacc att                                10473

<210>  6
<211>  10653
<212>  DNA
<213>  Artificial Sequence

<220>
<223>  Nucleotide sequence fof plasmid pSC_1b_5`AsiSI_lacZAmC_3`RsrII

<400>  6
cctgcaggta atacgactca ctatagccag cccccgattg ggggcgacac tccaccatag      60

atcactcccc tgtgaggaac tactgtcttc acgcagaaag cgtctagcca tggcgttagt     120

atgagtgtcg tgcagcctcc aggacccccc ctcccgggag agccatagtg gtctgcggaa     180

ccggtgagta caccggaatt gccaggacga ccgggtcctt tcttggatca acccgctcaa     240

tgcctggaga tttgggcgtg cccccgcgag actgctagcc gagtagtgtt gggtcgcgaa     300

aggccttgtg gtactgcctg atagggtgct tgcgagtgcc ccgggaggtc tcgtagaccg     360

tgcaccgttt aaacccccgt gctgctggaa gtcgatttca ggcttagggt aaccgtggac     420

ctcgaaaaca gacgcacaaa accaagttca atagaagggg gtacaaacca gtaccaccac     480

gaacaagcac ttctgtttcc ccggtgatgt cgtatagact gcttgcgtgg ttgaaagcga     540

cggatccgtt atccgcttat gtacttcgag aagcccagta ccacctcgga atcttcgatg     600

cgttgcgctc agcactcaac cccagagtgt agcttaggct gatgagtctg gacatccctc     660

accggtgacg gtggtccagg ctgcgttggc ggcctaccta tggctaacgc catgggacgc     720

tagttgtgaa caaggtgtga agagcctatt gagctacata agaatcctcc ggcccctgaa     780

tgcggctaat cccaacctcg gagcaggtgg tcacaaacca gtgattggcc tgtcgtaacg     840

cgcaagtccg tggcggaacc gactactttg ggtgtccgtg tttcctttta ttttattgtg     900

gctgcttatg gtgacaatca cagattgtta tcataaagcg aattggattg gccatccggt     960

gaaagtgaga ctcattatct atctgtttgc tggatccgct ccattgagtg tgtttactct    1020

aagtacaatt tcaacagtta tttcaatcag acaattgtat cataatggcg ggcccagaag    1080

acgccaaaaa cataaagaaa ggcccggcgc cattctatcc tctagaggat ggaaccgctg    1140

gagagcaact gcataaggct atgaagagat acgccctggt tcctggaaca attgctttta    1200

cagatgcaca tatcgaggtg aacatcacgt acgcggaata cttcgaaatg tccgttcggt    1260

tggcagaagc tatgaaacga tatgggctga atacaaatca cagaatcgtc gtatgcagtg 1320

aaaactctct tcaattcttt atgccggtgt tgggcgcgtt atttatcgga gttgcagttg 1380

cgcccgcgaa cgacatttat aatgaacgtg aattgctcaa cagtatgaac atttcgcagc 1440

ctaccgtagt gtttgtttcc aaaaaggggt tgcaaaaaat tttgaacgtg caaaaaaaat 1500

taccaataat ccagaaaatt attatcatgg attctaaaac ggattaccag ggatttcagt 1560

cgatgtacac gttcgtcaca tctcatctac ctcccggttt taatgaatac gattttgtac 1620

cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccagg acctcgtcgg 3600

ctggcaagcg ccccccgggg cgcgttcctt gacaccatgc acctgcggca gctcggacct 3660

ttacttggtc acgaggcatg ccgatgtcat tccggtgcgc cggcggggcg acagcagggg 3720

gagcctactc tcccccaggc ccgtctccta cttgaagggc tcttcgggcg gtccactgct 3780

ctgcccctcg gggcacgctg tgggcatctt tcgggctgcc gtgtgcaccc gaggggttgc 3840

gaaggcggtg gactttgtac ccgtcgagtc tatgggaacc actatgcggt ccccggtctt 3900

cacggacaac tcgtcccctc cggccgtacc gcagacattc caggtggccc atctacacgc 3960

ccctactggt agcggcaaga gcactaaggt gccggctgcg tatgcagccc aagggtataa 4020

ggtgcttgtc ctgaacccgt ccgtcgccgc caccctaggt ttcggggcgt atatgtctaa 4080

ggcacatggt atcgacccta acatcagaat cggggtaagg accatcacca cgggtgcccc 4140

catcacgtac tccacctatg gcaagtttct tgccgacggt ggttgctctg gggcgcccta 4200

tgacatcata atatgtgatg agtgccactc aactgactcg accactatcc tgggcatcgg 4260

cacagtcctg gaccaagcgg agacggctgg agcgcgactc gtcgtgctcg ccaccgctac 4320

gcctccggga tcggtcaccg tgccacatcc aaacatcgag gaggtggctc tgtccagcac 4380

tggagaaatc cccttttatg gcaaagccat ccccatcgag accatcaagg gggggaggca 4440

cctcattttc tgccattcca agaagaaatg tgatgagctc gccgcgaagc tgtccggcct 4500

cggactcaat gctgtagcat attaccgggg ccttgatgta tccgtcatac caactagcgg 4560
agacgtcatt gtcgtagcaa cggacgctct aatgacgggc tttaccggcg atttcgactc 4620
agtgatcgac tgcaatacat gtgtcaccca gacagtcgac ttcagcctgg acccgacctt 4680
caccattgag acgacgaccg tgccacaaga cgcggtgtca cgctcgcagc ggcgaggcag 4740
gactggtagg ggcaggatgg gcatttacag gtttgtgact ccaggagaac ggccctcggg 4800
catgttcgat tcctcggttc tgtgcgagtg ctatgacgcg ggctgtgctt ggtacgagct 4860
cacgcccgcc gagacctcag ttaggttgcg ggcttaccta aacacaccag ggttgcccgt 4920
ctgccaggac catctggagt tctgggagag cgtctttaca ggcctcaccc acatagacgc 4980
ccatttcttg tcccagacta agcaggcagg agacaacttc ccctacctgg tagcatacca 5040
ggctacggtg tgcgccaggg ctcaggctcc acctccatcg tgggaccaaa tgtggaagtg 5100
tctcatacgg ctaaagccta cgctgcacgg gccaacgccc ctgctgtata ggctgggagc 5160
cgttcaaaac gaggttacta ccacacaccc cataaccaaa tacatcatgg catgcatgtc 5220
ggctgacctg gaggtcgtca cgagcacctg ggtgctggta ggcggagtcc tagcagctct 5280
ggccgcgtat tgcctgacaa caggcagcgt ggtcattgtg ggcaggatca tcttgtccgg 5340
aaagccggcc atcattcccg acagggaagt cctttaccgg gagttcgatg agatggaaga 5400
gtgcgcctca cacctccctt acatcgaaca gggaatgcag ctcgccgaac aattcaaaca 5460
gaaggcaatc gggttgctgc aaacagccac caagcaagcg gaggctgctg ctcccgtggt 5520
ggaatccaag tggcggaccc tcgaagcctt ctgggcgaag catatgtgga atttcatcag 5580
cgggatacaa tatttagcag gcttgtccac tctgcctggc aaccccgcga tagcatcact 5640
gatggcattc acagcctcta tcaccagccc gctcaccacc caacataccc tcctgtttaa 5700
catcctgggg ggatgggtgg ccgcccaact tgctcctccc agcgctgctt ctgctttcgt 5760
aggcgccggc atcgctggag cggctgttgg cagcataggc cttgggacgg tgcttgtgga 5820
tattttggca ggttatggag caggggtggc aggcgcgctc gtggccttta aggtcatgag 5880
cggcgagatg ccctccaccg aggacctggt taacctactc cctgctatcc tctcccctgg 5940
cgccctagtc gtcggggtcg tgtgcgcagc gatactgcgt cggcacgtgg gcccagggga 6000
gggggctgtg cagtggatga accggctgat agcgttcgct tcgcggggta accacgtctc 6060
ccccacgcac tatgtgcctg agagcgacgc tgcagcacgt gtcactcaga tcctctctag 6120

tcttaccatc actcagctgc tgaagaggct tcaccagtgg atcaacgagg actgctccac 6180

gccatgctcc ggctcgtggc taagagatgt ttgggattgg atatgcacgg tgttgactga 6240

tttcaagacc tggctccagt ccaagctcct gccgcgattg ccgggagtcc ccttcttctc 6300

atgtcaacgt gggtacaagg gagtctggcg gggcgacggc atcatgcaaa ccacctgccc 6360

atgtggagca cagatcaccg gacatgtgaa aaacggttcc atgaggatcg tggggcctag 6420

gacctgtagt aacacgtggc atggaacatt ccccattaac gcgtacacca cgggcccctg 6480

cacgccctcc ccggcgccaa attattctag ggcgctgtgg cgggtggctg ctgaggagta 6540

cgtggaggtt acgcgggtgg gggatttcca ctacgtgacg ggcatgacca ctgacaacgt 6600

aaagtgcccg tgtcaggttc cggcccccga attcttcaca gaagtggatg gggtgcggtt 6660

gcacaggtac gctccagcgt gcaaacccct cctacgggag gaggtcacat tcctggtcgg 6720

gctcaatcaa tacctggttg ggtcacagct cccatgcgag cccgaaccgg acgtagcagt 6780

gctcacttcc atgctcaccg acccctccca cattacggcg gagacggcta agcgtaggct 6840

ggccagggga tctcccccct ccttggccag ctcatcagct agccagctgt ctgcgccttc 6900

cttgaaggca acatgcacta cccgtcatga ctccccggac gctgacctca tcgaggccaa 6960

cctcctgtgg cggcaggaga tgggcgggaa catcacccgc gtggagtcag aaaataaggt 7020

agtaattttg gactctttcg agccgctcca agcggaggag gatgagaggg aagtatccgt 7080

tccggcggag atcctgcgga ggtccaggaa attccctcga gcgatgccca tatgggcacg 7140

cccggattac aaccctccac tgttagagtc ctggaaggac ccggactacg tccctccagt 7200

ggtacacggg tgtccattgc cgcctgccaa ggcccctccg ataccacctc cacggaggaa 7260

gaggacggtt gtcctgtcag aatctaccgt gtcttctgcc ttggcggagc tcgccacaaa 7320

gaccttcggc agctccgaat cgtcggccgt cgacagcggc acggcaacgg cctctcctga 7380

ccagccctcc gacgacggcg acgcgggatc cgacgttgag tcgtactcct ccatgccccc 7440

ccttgagggg gagccggggg atcccgatct cagcgacggg tcttggtcta ccgtaagcga 7500

ggaggctagt gaggacgcga tcgcgtgagc gcaacgcaat taatgtgagt tagctcactc 7560

attaggcacc ccaggcttta cactttatgc ttccggctcg tatgttgtgt ggaattgtga 7620

gcggataaca atttcacaca ggaaacagct atgaccatga ttacgccaag cttgcatgcc 7680

tgcaggtcga ctctagagga tccccgggta ccggtcgcca ccatggctct ttcaaacaag 7740

tttatcggag atgacatgaa aatgacctac catatggatg gctgtgtcaa tgggcattac 7800

tttaccgtca aaggtgaagg cagcgggaag ccatacgaag ggacgcagac ctcgactttt 7860

aaagtcacca tggccaacgg tgggcccctt gcattctcct ttgacatact atctacagtg 7920

ttcatgtatg gaaatcgatg ctttactgcg tatcctacca gtatgcccga ctatttcaaa 7980

caagcatttc ctgacggaat gtcatatgaa aggactttta cctatgaaga tggaggagtt 8040

gctacagcca gttgggaaat aagccttaaa ggcaactgct ttgagcacaa atccacgttt 8100

catggagtga actttcctgc tgatggacct gtgatggcga agatgacaac tggttgggac 8160

ccatcttttg agaaaatgac tgtctgcgat ggaatattga agggtgatgt caccgcgttc 8220

ctcatgctgc aaggaggtgg caattacaga tgccaattcc acacttctta caagacaaaa 8280

aaaccggtga cgatgccacc aaaccatgcg gtggaacatc gcattgcgag gaccgacctt 8340

gacaaaggtg gcaacagtgt tcagctgacg gagcacgctg ttgcacatat aacctctgtt 8400

gtccctttct gaagcggccg cgactctaga attccaactc ggtccggcta aacactccag 8460

gccaataggc catcctgttt ttttcccttt ttttttttct tttttttttt tttttttttt 8520

tttttttttt tttctccttt ttttttcctc tttttttcct tttctttcct ttggtggctc 8580

catcttagcc ctagtcacgg ctagctgtga aaggtccgtg agccgcttga ctgcagagag 8640

tgctgatact ggcctctctg cagatcaagt actactagta gaggcggttt gcgtattggg 8700

cgctcttccg cttcctcgct cactgactcg ctgcgctcgg tcgttcggct gcggcgagcg 8760

gtatcagctc actcaaaggc ggtaatacgg ttatccacag aatcagggga taacgcagga 8820

aagaacatgt gagcaaaagg ccagcaaaag gccaggaacc gtaaaaaggc cgcgttgctg 8880

gcgtttttcc ataggctccg cccccctgac gagcatcaca aaaatcgacg ctcaagtcag 8940

aggtggcgaa acccgacagg actataaaga taccaggcgt ttccccctgg aagctccctc 9000

gtgcgctctc ctgttccgac cctgccgctt accggatacc tgtccgcctt tctcccttcg 9060

ggaagcgtgg cgctttctca tagctcacgc tgtaggtatc tcagttcggt gtaggtcgtt 9120

cgctccaagc tgggctgtgt gcacgaaccc cccgttcagc ccgaccgctg cgccttatcc 9180

ggtaactatc gtcttgagtc caacccggta agacacgact tatcgccact ggcagcagcc 9240

actggtaaca ggattagcag agcgaggtat gtaggcggtg ctacagagtt cttgaagtgg 9300

tggcctaact acggctacac tagaaggaca gtatttggta tctgcgctct gctgaagcca 9360

gttaccttcg gaaaaagagt tggtagctct tgatccggca aacaaaccac cgctggtagc 9420

ggtggttttt ttgtttgcaa gcagcagatt acgcgcagaa aaaaaggatc tcaagaagat 9480

cctttgatct tttctacggg gtctgacgct cagtggaacg aaaactcacg ttaagggatt 9540

ttggtcatga gattatcaaa aaggatcttc acctagatcc ttttaaatta aaaatgaagt 9600

tttaaatcaa tctaaagtat atatgagtaa acttggtctg acagttacca atgcttaatc 9660

agtgaggcac ctatctcagc gatctgtcta tttcgttcat ccatagttgc ctgactcccc 9720

gtcgtgtaga taactacgat acgggagggc ttaccatctg gccccagtgc tgcaatgata 9780

ccgcgagacc cacgctcacc ggctccagat ttatcagcaa taaaccagcc agccggaagg 9840

gccgagcgca gaagtggtcc tgcaacttta tccgcctcca tccagtctat taattgttgc 9900

cgggaagcta gagtaagtag ttcgccagtt aatagtttgc gcaacgttgt tgccattgct 9960

acaggcatcg tggtgtcacg ctcgtcgttt ggtatggctt cattcagctc cggttcccaa 10020

cgatcaaggc gagttacatg atcccccatg ttgtgcaaaa aagcggttag ctccttcggt 10080

cctccgatcg ttgtcagaag taagttggcc gcagtgttat cactcatggt tatggcagca 10140

ctgcataatt ctcttactgt catgccatcc gtaagatgct tttctgtgac tggtgagtac 10200

tcaaccaagt cattctgaga atagtgtatg cggcgaccga gttgctcttg cccggcgtca 10260

atacgggata ataccgcgcc acatagcaga actttaaaag tgctcatcat tggaaaacgt 10320

tcttcggggc gaaaactctc aaggatctta ccgctgttga gatccagttc gatgtaaccc 10380

actcgtgcac ccaactgatc ttcagcatct tttactttca ccagcgtttc tgggtgagca 10440

aaaacaggaa ggcaaaatgc cgcaaaaaag ggaataaggg cgacacggaa atgttgaata 10500

ctcatactct tcctttttca atattattga agcatttatc agggttattg tctcatgagc 10560

ggatacatat ttgaatgtat ttagaaaaat aaacaaatag gggttccgcg cacatttccc 10620

cgaaaagtgc cacctgacgt ctaagaaacc att 10653

<210> 7
<211> 11509
<212> DNA
<213> Artificial Sequence

<220>
<223> Nucleotide sequence of plasmid pSS-1_1a_NS5B_5`AsiSI_3`RsrII

<400>  7
cctgcaggta atacgactca ctatagccag cccccgattg ggggcgacac tccaccatag      60

atcactcccc tgtgaggaac tactgtcttc acgcagaaag cgtctagcca tggcgttagt     120

atgagtgtcg tgcagcctcc aggacccccc ctcccgggag agccatagtg gtctgcggaa     180

ccggtgagta caccggaatt gccaggacga ccgggtcctt tcttggatca acccgctcaa     240

tgcctggaga tttgggcgtg cccccgcgag actgctagcc gagtagtgtt gggtcgcgaa     300

aggccttgtg gtactgcctg atagggtgct tgcgagtgcc ccgggaggtc tcgtagaccg     360

tgcaccgttt aaacccccgt gctgctggaa gtcgatttca ggcttagggt aaccgtggac     420

ctcgaaaaca gacgcacaaa accaagttca atagaagggg gtacaaacca gtaccaccac     480

gaacaagcac ttctgtttcc ccggtgatgt cgtatagact gcttgcgtgg ttgaaagcga     540

cggatccgtt atccgcttat gtacttcgag aagcccagta ccacctcgga atcttcgatg     600

cgttgcgctc agcactcaac cccagagtgt agcttaggct gatgagtctg gacatccctc     660

accggtgacg gtggtccagg ctgcgttggc ggcctaccta tggctaacgc catgggacgc     720

tagttgtgaa caaggtgtga agagcctatt gagctacata agaatcctcc ggcccctgaa     780

tgcggctaat cccaacctcg gagcaggtgg tcacaaacca gtgattggcc tgtcgtaacg     840

cgcaagtccg tggcggaacc gactactttg ggtgtccgtg tttcctttta ttttattgtg     900

gctgcttatg gtgacaatca cagattgtta tcataaagcg aattggattg gccatccggt     960

gaaagtgaga ctcattatct atctgtttgc tggatccgct ccattgagtg tgtttactct    1020

aagtacaatt tcaacagtta tttcaatcag acaattgtat cataatggcg ggcccagaag    1080

acgccaaaaa cataaagaaa ggcccggcgc cattctatcc tctagaggat ggaaccgctg    1140

gagagcaact gcataaggct atgaagagat acgccctggt tcctggaaca attgctttta    1200

cagatgcaca tatcgaggtg aacatcacgt acgcggaata cttcgaaatg tccgttcggt    1260

tggcagaagc tatgaaacga tatgggctga atacaaatca cagaatcgtc gtatgcagtg    1320

aaaactctct tcaattcttt atgccggtgt tgggcgcgtt atttatcgga gttgcagttg    1380

cgcccgcgaa cgacatttat aatgaacgtg aattgctcaa cagtatgaac atttcgcagc    1440

ctaccgtagt gtttgtttcc aaaaaggggt tgcaaaaaat tttgaacgtg caaaaaaaat    1500

taccaataat ccagaaaatt attatcatgg attctaaaac ggattaccag ggatttcagt    1560

cgatgtacac gttcgtcaca tctcatctac ctcccggttt taatgaatac gattttgtac    1620

cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccaag accttgtggg 3600

ctggcccgct cctcaaggtt cccgctcatt gacaccctgt acctgcggct cctcggacct 3660

ttacctggtc acgaggcacg ccgatgtcat tcccgtgcgc cggcgaggtg atagcagggg 3720

tagcctgctt tcgccccggc ccatttccta cttgaaaggc tcctcggggg gtccgctgtt 3780

gtgccccgcg ggacacgccg tgggcctatt cagggccgcg gtgtgcaccc gtggagtggc 3840

taaagcggtg gactttatcc ctgtggagaa cctagggaca accatgagat ccccggtgtt 3900

cacggacaac tcctctccac cagcagtgcc ccagagcttc caggtggccc acctgcatgc 3960

tcccaccggc agcggtaaga gcaccaaggt cccggctgcg tacgcagccc agggctacaa 4020

ggtgttggtg ctcaacccct ctgttgctgc aacgctgggc tttggtgctt acatgtccaa 4080

ggcccatggg gttgatccta atatcaggac cggggtgaga acaattacca ctggcagccc 4140

catcacgtac tccacctacg gcaagttcct tgccgacggc gggtgctcag gaggtgctta 4200

tgacataata atttgtgacg agtgccactc cacggatgcc acatccatct tgggcatcgg 4260

cactgtcctt gaccaagcag agactgcggg ggcgagactg gttgtgctcg ccactgctac 4320

ccctccgggc tccgtcactg tgtcccatcc taacatcgag gaggttgctc tgtccaccac 4380

cggagagatc cccttttacg gcaaggctat ccccctcgag gtgatcaagg ggggaagaca 4440

tctcatcttc tgccactcaa agaagaagtg cgacgagctc gccgcgaagc tggtcgcatt 4500

gggcatcaat gccgtggcct actaccgcgg tcttgacgtg tctgtcatcc cgaccagcgg 4560

cgatgttgtc gtcgtgtcga ccgatgctct catgactggc tttaccggcg acttcgactc 4620

tgtgatagac tgcaacacgt gtgtcactca gacagtcgat ttcagccttg accctacctt 4680

taccattgag acaaccacgc tcccccagga tgctgtctcc aggactcaac gccggggcag 4740

gactggcagg gggaagccag gcatctatag atttgtggca ccgggggagc gcccctccgg 4800

catgttcgac tcgtccgtcc tctgtgagtg ctatgacgcg ggctgtgctt ggtatgagct 4860

cacgcccgcc gagactacag ttaggctacg agcgtacatg aacaccccgg ggcttcccgt 4920

gtgccaggac catcttgaat tttgggaggg cgtctttacg ggcctcactc atatagatgc 4980

ccacttttta tcccagacaa agcagagtgg ggagaacttt ccttacctgg tagcgtacca 5040

agccaccgtg tgcgctaggg ctcaagcccc tcccccatcg tgggaccaga tgtggaagtg 5100

tttgatccgc cttaaaccca ccctccatgg gccaacaccc ctgctataca gactgggcgc 5160

tgttcagaat gaagtcaccc tgacgcaccc aatcaccaaa tacatcatga catgcatgtc 5220

ggccgacctg gaggtcgtca cgagcacctg ggtgctcgtt ggcggcgtcc tggctgctct 5280

ggccgcgtat tgcctgtcaa caggctgcgt ggtcatagtg ggcaggatcg tcttgtccgg 5340

gaagccggca attatacctg acagggaggt tctctaccag gagttcgatg agatggaaga 5400

gtgctctcag cacttaccgt acatcgagca agggatgatg ctcgctgagc agttcaagca 5460

gaaggccctc ggcctcctgc agaccgcgtc ccgccatgca gaggttatca cccctgctgt 5520

ccagaccaac tggcagaaac tcgaggtctt ttgggcgaag cacatgtgga atttcatcag 5580

tgggatacaa tacttggcgg gcctgtcaac gctgcctggt aaccccgcca ttgcttcatt 5640

gatggctttt acagctgccg tcaccagccc actaaccact ggccaaaccc tcctcttcaa 5700

catattgggg gggtgggtgg ctgcccagct cgccgccccc ggtgccgcta ctgcctttgt 5760

gggtgctggc ctagctggcg ccgccatcgg cagcgttgga ctggggaagg tcctcgtgga 5820

cattcttgca gggtatggcg cgggcgtggc gggagctctt gtagcattca agatcatgag 5880

cggtgaggtc ccctccacgg aggacctggt caatctgctg cccgccatcc tctcgcctgg 5940

agcccttgta gtcggtgtgg tctgcgcagc aatactgcgc cggcacgttg gcccgggcga 6000

gggggcagtg caatggatga accggctaat agccttcgcc tcccggggga accatgtttc 6060

ccccacgcac tacgtgccgg agagcgatgc agccgcccgc gtcactgcca tactcagcag 6120

cctcactgta acccagctcc tgaggcgact gcatcagtgg ataagctcgg agtgtaccac 6180

tccatgctcc ggttcctggc taagggacat ctgggactgg atatgcgagg tgctgagcga 6240

ctttaagacc tggctgaaag ccaagctcat gccacaactg cctgggattc cctttgtgtc 6300

ctgccagcgc gggtataggg gggtctggcg aggagacggc attatgcaca ctcgctgcca 6360

ctgtggagct gagatcactg gacatgtcaa aaacgggacg atgaggatcg tcggtcctag 6420

gacctgcagg aacatgtgga gtgggacgtt ccccattaac gcctacacca cgggcccctg 6480

tactcccctt cctgcgccga actataagtt cgcgctgtgg agggtgtctg cagaggaata 6540

cgtggagata aggcgggtgg gggacttcca ctacgtatcg ggtatgacta ctgacaatct 6600

taaatgcccg tgccagatcc catcgcccga attcttcaca gaattggacg gggtgcgcct 6660

acacaggttt gcgccccctt gcaagccctt gctgcgggag gaggtatcat tcagagtagg 6720

actccacgag tacccggtgg ggtcgcaatt accttgcgag cccgaaccgg acgtagccgt 6780

gttgacgtcc atgctcactg atccctccca tataacagca gaggcggccg ggagaaggtt 6840

ggcgagaggg tcaccccctt ctatggccag ctcctcggct atccagctgt ccgctccatc 6900

tctcaaggca acttgcaccg ccaaccatga ctcccctgac gccgagctca tagaggctaa 6960

cctcctgtgg aggcaggaga tgggcggcaa catcaccagg gttgagtcag agaacaaagt 7020

ggtgattctg gactccttcg atccgcttgt ggcagaggag gatgagcggg aggtctccgt 7080

acctgcagaa attctgcgga agtctcggag attcgcccgg gccctgcccg tctgggcgcg 7140

gccggactac aaccccccgc tagtagagac gtggaaaaag cctgactacg aaccacctgt 7200

ggtccatggc tgcccgctac cacctccacg gtcccctcct gtgcctccgc ctcggaaaaa 7260

gcgtacggtg gtcctcaccg aatcaaccct atctactgcc ttggccgagc ttgccaccaa 7320

aagttttggc agctcctcaa cttccggcat tacgggcgac aatacgacaa catcctctga 7380

gcccgcccct tctggctgcc cccccgactc cgacgttgag tcctattctt ccatgccccc 7440

cctggagggg gagcctgggg atccggatct cagcgacggg tcatggtcga cggtcagtag 7500

tggggccgac acggaagatg cgatcgcctg ctcaatgtct tattcctgga caggcgcact 7560

cgtcaccccg tgcgctgcgg aagaacaaaa actgcccatc aacgcactga gcaactcgtt 7620

gctacgccat cacaatctgg tgtattccac cacttcacgc agtgcttgcc aaaggcagaa 7680

gaaagtcaca tttgacagac tgcaagttct ggacagccat taccaggacg tgctcaagga 7740

ggtcaaagca gcggcgtcaa aagtgaaggc taacttgcta tccgtagagg aagcttgcag 7800

cctgacgccc ccacattcag ccaaatccaa gtttggctat ggggcaaaag acgtccgttg 7860

ccatgccaga aaggccgtag cccacatcaa ctccgtgtgg aaagaccttc tggaagacag 7920

tgtaacacca attgacacta ccatcatggc caagaacgag gttttctgcg ttcagcctga 7980

gaaggggggt cgtaagccag ctcgtctcat cgtgttcccc gacctgggcg tgcgcgtgtg 8040

cgagaagatg gccctgtacg acgtggttag caagctcccc ctggccgtga tgggaagctc 8100

ctacggattc caatactcac caggacagcg ggttgaattc ctcgtgcaag cgtggaagtc 8160

caagaagacc ccgatggggt tctcgtatga tacccgctgt tttgactcca cagtcactga 8220

gagcgacatc cgtacggagg aggcaattta ccaatgttgt gacctggacc cccaagcccg 8280

cgtggccatc aagtccctca ctgagaggct ttatgttggg ggccctctta ccaattcaag 8340

gggggaaaac tgcggctacc gcaggtgccg cgcgagcggc gtactgacaa ctagctgtgg 8400

taacaccctc acttgctaca tcaaggcccg ggcagcctgt cgagccgcag ggctccagga 8460

ctgcaccatg ctcgtgtgtg gcgacgactt agtcgttatc tgtgaaagtg cgggggtcca 8520

ggaggacgcg gcgagcctga gagccttcac ggaggctatg accaggtact ccgccccccc 8580

cggggacccc ccacaaccag aatacgactt ggagcttata acatcatgct cctccaacgt 8640

gtcagtcgcc cacgacggcg ctggaaagag ggtctactac cttacccgtg accctacaac 8700

ccccctcgcg agagccgcgt gggagacagc aagacacact ccagtcaatt cctggctagg 8760

caacataatc atgtttgccc ccacactgtg ggcgaggatg atactgatga cccatttctt 8820

tagcgtcctc atagccaggg atcagcttga acaggctctt aactgtgaga tctacggagc 8880

ctgctactcc atagaaccac tggatctacc tccaatcatt caaagactcc atggcctcag 8940

cgcattttca ctccacagtt actctccagg tgaaatcaat agggtggccg catgcctcag 9000

aaaacttggg gtcccgccct tgcgagcttg gagacaccgg gcccggagcg tccgcgctag 9060

gcttctgtcc agaggaggca gggctgccat atgtggcaag tacctcttca actgggcagt 9120

aagaacaaag ctcaaactca ctccaatagc ggccgctggc cggctggact tgtccggttg 9180

gttcacggct ggctacagcg ggggagacat ttatcacagc gtgtctcatg cccggccccg 9240

ctggttctgg ttttgcctac tcctgctcgc tgcaggggta ggcatctacc tcctccccaa 9300

ccgatgacgg tccgggtaaa cactccggcc tcttaagcca tttcctgttt tttttttttt 9360

tttttttttt tttttctttt tttttttctt tcctttcctt ctttttttcc tttctttttc 9420

ccttctttaa tggtggctcc atcttagccc tagtcacggc tagctgtgaa aggtccgtga 9480

gccgcttgac tgcagagagt gctgatactg gcctctctgc agatcaagta ctagtagagg 9540

cggtttgcgt attgggcgct cttccgcttc ctcgctcact gactcgctgc gctcggtcgt 9600

tcggctgcgg cgagcggtat cagctcactc aaaggcggta atacggttat ccacagaatc 9660

aggggataac gcaggaaaga acatgtgagc aaaaggccag caaaaggcca ggaaccgtaa 9720

aaaggccgcg ttgctggcgt ttttccatag gctccgcccc cctgacgagc atcacaaaaa 9780

tcgacgctca agtcagaggt ggcgaaaccc gacaggacta taaagatacc aggcgtttcc 9840

ccctggaagc tccctcgtgc gctctcctgt tccgaccctg ccgcttaccg gatacctgtc 9900

cgcctttctc ccttcgggaa gcgtggcgct ttctcatagc tcacgctgta ggtatctcag 9960

ttcggtgtag gtcgttcgct ccaagctggg ctgtgtgcac gaaccccccg ttcagcccga 10020

ccgctgcgcc ttatccggta actatcgtct tgagtccaac ccggtaagac acgacttatc 10080

gccactggca gcagccactg gtaacaggat tagcagagcg aggtatgtag gcggtgctac 10140

agagttcttg aagtggtggc ctaactacgg ctacactaga aggacagtat ttggtatctg 10200

cgctctgctg aagccagtta ccttcggaaa aagagttggt agctcttgat ccggcaaaca 10260

aaccaccgct ggtagcggtg gtttttttgt ttgcaagcag cagattacgc gcagaaaaaa 10320

aggatctcaa gaagatcctt tgatcttttc tacggggtct gacgctcagt ggaacgaaaa 10380

ctcacgttaa gggattttgg tcatgagatt atcaaaaagg atcttcacct agatcctttt 10440

aaattaaaaa tgaagtttta aatcaatcta aagtatatat gagtaaactt ggtctgacag 10500

ttaccaatgc ttaatcagtg aggcacctat ctcagcgatc tgtctatttc gttcatccat 10560

agttgcctga ctccccgtcg tgtagataac tacgatacgg gagggcttac catctggccc 10620

cagtgctgca atgataccgc gagacccacg ctcaccggct ccagatttat cagcaataaa 10680

ccagccagcc ggaagggccg agcgcagaag tggtcctgca actttatccg cctccatcca 10740

gtctattaat tgttgccggg aagctagagt aagtagttcg ccagttaata gtttgcgcaa 10800

cgttgttgcc attgctacag gcatcgtggt gtcacgctcg tcgtttggta tggcttcatt 10860

cagctccggt tcccaacgat caaggcgagt tacatgatcc cccatgttgt gcaaaaaagc 10920

ggttagctcc ttcggtcctc cgatcgttgt cagaagtaag ttggccgcag tgttatcact 10980

catggttatg gcagcactgc ataattctct tactgtcatg ccatccgtaa gatgcttttc 11040

tgtgactggt gagtactcaa ccaagtcatt ctgagaatag tgtatgcggc gaccgagttg 11100

ctcttgcccg gcgtcaatac gggataatac cgcgccacat agcagaactt taaaagtgct 11160

catcattgga aaacgttctt cggggcgaaa actctcaagg atcttaccgc tgttgagatc 11220

cagttcgatg taacccactc gtgcacccaa ctgatcttca gcatctttta ctttcaccag 11280

cgtttctggg tgagcaaaaa caggaaggca aaatgccgca aaaaagggaa taagggcgac 11340

acggaaatgt tgaatactca tactcttcct ttttcaatat tattgaagca tttatcaggg 11400

ttattgtctc atgagcggat acatatttga atgtatttag aaaaataaac aaataggggt 11460

tccgcgcaca tttccccgaa aagtgccacc tgacgtctaa gaaaccatt 11509

<210> 8
<211> 10644
<212> DNA
<213> Artificial Sequence

<220>
<223> Nucleotide sequence of plasmid pSS-1_1a_5`AsiSI_lacZAmC_3`RsrII

<400> 8
cctgcaggta atacgactca ctatagccag cccccgattg ggggcgacac tccaccatag 60

atcactcccc tgtgaggaac tactgtcttc acgcagaaag cgtctagcca tggcgttagt 120

atgagtgtcg tgcagcctcc aggacccccc ctcccgggag agccatagtg gtctgcggaa 180

ccggtgagta caccggaatt gccaggacga ccgggtcctt tcttggatca acccgctcaa 240

tgcctggaga tttgggcgtg cccccgcgag actgctagcc gagtagtgtt gggtcgcgaa 300

aggccttgtg gtactgcctg atagggtgct tgcgagtgcc ccgggaggtc tcgtagaccg 360

tgcaccgttt aaacccccgt gctgctggaa gtcgatttca ggcttagggt aaccgtggac 420

ctcgaaaaca gacgcacaaa accaagttca atagaagggg gtacaaacca gtaccaccac 480

gaacaagcac ttctgtttcc ccggtgatgt cgtatagact gcttgcgtgg ttgaaagcga 540

cggatccgtt atccgcttat gtacttcgag aagcccagta ccacctcgga atcttcgatg 600

cgttgcgctc agcactcaac cccagagtgt agcttaggct gatgagtctg gacatccctc 660

accggtgacg gtggtccagg ctgcgttggc ggcctaccta tggctaacgc catgggacgc 720

tagttgtgaa caaggtgtga agagcctatt gagctacata agaatcctcc ggcccctgaa 780

tgcggctaat cccaacctcg gagcaggtgg tcacaaacca gtgattggcc tgtcgtaacg 840

cgcaagtccg tggcggaacc gactactttg ggtgtccgtg tttcctttta ttttattgtg 900

gctgcttatg gtgacaatca cagattgtta tcataaagcg aattggattg gccatccggt 960

gaaagtgaga ctcattatct atctgtttgc tggatccgct ccattgagtg tgtttactct 1020

aagtacaatt tcaacagtta tttcaatcag acaattgtat cataatggcg ggcccagaag 1080

acgccaaaaa cataaagaaa ggcccggcgc cattctatcc tctagaggat ggaaccgctg 1140

gagagcaact gcataaggct atgaagagat acgccctggt tcctggaaca attgctttta 1200

cagatgcaca tatcgaggtg aacatcacgt acgcggaata cttcgaaatg tccgttcggt 1260

tggcagaagc tatgaaacga tatgggctga atacaaatca cagaatcgtc gtatgcagtg 1320

aaaactctct tcaattcttt atgccggtgt tgggcgcgtt atttatcgga gttgcagttg 1380

cgcccgcgaa cgacatttat aatgaacgtg aattgctcaa cagtatgaac atttcgcagc 1440

ctaccgtagt gtttgtttcc aaaaaggggt tgcaaaaaat tttgaacgtg caaaaaaaat 1500

taccaataat ccagaaaatt attatcatgg attctaaaac ggattaccag ggatttcagt 1560

cgatgtacac gttcgtcaca tctcatctac ctcccggttt taatgaatac gattttgtac 1620

cagagtcctt tgatcgtgac aaaacaattg cactgataat gaattcctct ggatctactg 1680

ggttacctaa gggtgtggcc cttccgcata gaactgcctg cgtcagattc tcgcatgcca 1740

gagatcctat ttttggcaat caaatcattc cggatactgc gattttaagt gttgttccat 1800

tccatcacgg ttttggaatg tttactacac tcggatattt gatatgtgga tttcgagtcg 1860

tcttaatgta tagatttgaa gaagagctgt ttttacgatc ccttcaggat tacaaaattc 1920

aaagtgcgtt gctagtacca accctatttt cattcttcgc caaaagcact ctgattgaca 1980

aatacgattt atctaattta cacgaaattg cttctggggg cgcacctctt tcgaaagaag 2040

tcggggaagc ggttgcaaaa cgcttccatc ttccagggat acgacaagga tatgggctca 2100

ctgagactac atcagctatt ctgattacac ccgaggggga tgataaaccg ggcgcggtcg 2160

gtaaagttgt tccatttttt gaagcgaagg ttgtggatct ggataccggg aaaacgctgg 2220

gcgttaatca gagaggcgaa ttatgtgtca gaggacctat gattatgtcc ggttatgtaa 2280

acaatccgga agcgaccaac gccttgattg acaaggatgg atggctacat tctggagaca 2340

tagcttactg ggacgaagac gaacacttct tcatagttga ccgcttgaag tctttaatta 2400

aatacaaagg atatcaggtg gcccccgctg aattggaatc gatattgtta caacacccca 2460

acatcttcga cgcgggcgtg gcaggtcttc ccgacgatga cgccggtgaa cttcccgccg 2520

ccgttgttgt tttggagcac ggaaagacga tgacggaaaa agagatcgtg gattacgtcg 2580

ccagtcaagt aacaaccgcg aaaaagttgc gcggaggagt tgtgtttgtg gacgaagtac 2640

cgaaaggtct taccggaaaa ctcgacgcaa gaaaaatcag agagatcctc ataaaggcca 2700

agaagggcgg aaagtccaaa ttgtaagcgg ccgcgttgtt aaacagacca caacggtttc 2760

cctctagcgg gatcaattcc gccccccccc cctaacgtta ctggccgaag ccgcttggaa 2820

taaggccggt gtgcgtttgt ctatatgtta ttttccacca tattgccgtc ttttggcaat 2880

gtgagggccc ggaaacctgg ccctgtcttc ttgacgagca ttcctagggg tctttcccct 2940

ctcgccaaag gaatgcaagg tctgttgaat gtcgtgaagg aagcagttcc tctggaagct 3000

tcttgaagac aaacaacgtc tgtagcgacc ctttgcaggc agcggaaccc cccacctggc 3060

gacaggtgcc tctgcggcca aaagccacgt gtataagata cacctgcaaa ggcggcacaa 3120

ccccagtgcc acgttgtgag ttggatagtt gtggaaagag tcaaatggct ctcctcaagc 3180

gtattcaaca aggggctgaa ggatgcccag aaggtacccc attgtatggg atctgatctg 3240

gggcctcggt gcacatgctt tacatgtgtt tagtcgaggt taaaaaaacg tctaggcccc 3300

ccgaaccacg gggacgtggt tttcctttga aaaacacgat aataccatgg cgcctattac 3360

ggcctactcc caacagacgc gaggcctact tggctgcatc atcactagcc tcacaggccg 3420

ggacaggaac caggtcgagg gggaggtcca agtggtctcc accgcaacac aatctttcct 3480

ggcgacctgc gtcaatggcg tgtgttggac tgtctatcat ggtgccggct caaagaccct 3540

tgccggccca aagggcccaa tcacccaaat gtacaccaat gtggaccaag accttgtggg 3600

ctggcccgct cctcaaggtt cccgctcatt gacaccctgt acctgcggct cctcggacct 3660

ttacctggtc acgaggcacg ccgatgtcat tcccgtgcgc cggcgaggtg atagcagggg 3720

tagcctgctt tcgccccggc ccatttccta cttgaaaggc tcctcggggg gtccgctgtt 3780

gtgccccgcg ggacacgccg tgggcctatt cagggccgcg gtgtgcaccc gtggagtggc 3840

taaagcggtg gactttatcc ctgtggagaa cctagggaca accatgagat ccccggtgtt 3900

cacggacaac tcctctccac cagcagtgcc ccagagcttc caggtggccc acctgcatgc 3960

tcccaccggc agcggtaaga gcaccaaggt cccggctgcg tacgcagccc agggctacaa 4020

ggtgttggtg ctcaacccct ctgttgctgc aacgctgggc tttggtgctt acatgtccaa 4080

ggcccatggg gttgatccta atatcaggac cggggtgaga acaattacca ctggcagccc 4140

catcacgtac tccacctacg gcaagttcct tgccgacggc gggtgctcag gaggtgctta 4200

tgacataata atttgtgacg agtgccactc cacggatgcc acatccatct tgggcatcgg 4260

cactgtcctt gaccaagcag agactgcggg ggcgagactg gttgtgctcg ccactgctac 4320

ccctccgggc tccgtcactg tgtcccatcc taacatcgag gaggttgctc tgtccaccac 4380

cggagagatc cccttttacg gcaaggctat ccccctcgag gtgatcaagg ggggaagaca 4440

tctcatcttc tgccactcaa agaagaagtg cgacgagctc gccgcgaagc tggtcgcatt 4500

gggcatcaat gccgtggcct actaccgcgg tcttgacgtg tctgtcatcc cgaccagcgg 4560

cgatgttgtc gtcgtgtcga ccgatgctct catgactggc tttaccggcg acttcgactc 4620

tgtgatagac tgcaacacgt gtgtcactca gacagtcgat ttcagccttg accctacctt 4680

taccattgag acaaccacgc tcccccagga tgctgtctcc aggactcaac gccggggcag 4740

gactggcagg gggaagccag gcatctatag atttgtggca ccgggggagc gcccctccgg 4800

catgttcgac tcgtccgtcc tctgtgagtg ctatgacgcg ggctgtgctt ggtatgagct 4860

cacgcccgcc gagactacag ttaggctacg agcgtacatg aacaccccgg ggcttcccgt 4920

gtgccaggac catcttgaat tttgggaggg cgtctttacg ggcctcactc atatagatgc 4980

ccacttttta tcccagacaa agcagagtgg ggagaacttt ccttacctgg tagcgtacca 5040

agccaccgtg tgcgctaggg ctcaagcccc tcccccatcg tgggaccaga tgtggaagtg 5100

tttgatccgc cttaaaccca ccctccatgg gccaacaccc ctgctataca gactgggcgc 5160

tgttcagaat gaagtcaccc tgacgcaccc aatcaccaaa tacatcatga catgcatgtc 5220

ggccgacctg gaggtcgtca cgagcacctg ggtgctcgtt ggcggcgtcc tggctgctct 5280

ggccgcgtat tgcctgtcaa caggctgcgt ggtcatagtg ggcaggatcg tcttgtccgg 5340

gaagccggca attatacctg acagggaggt tctctaccag gagttcgatg agatggaaga 5400

gtgctctcag cacttaccgt acatcgagca agggatgatg ctcgctgagc agttcaagca 5460

gaaggccctc ggcctcctgc agaccgcgtc ccgccatgca gaggttatca cccctgctgt 5520

ccagaccaac tggcagaaac tcgaggtctt ttgggcgaag cacatgtgga atttcatcag 5580

tgggatacaa tacttggcgg gcctgtcaac gctgcctggt aaccccgcca ttgcttcatt 5640

gatggctttt acagctgccg tcaccagccc actaaccact ggccaaaccc tcctcttcaa 5700

catattgggg gggtgggtgg ctgcccagct cgccgccccc ggtgccgcta ctgcctttgt 5760

gggtgctggc ctagctggcg ccgccatcgg cagcgttgga ctggggaagg tcctcgtgga 5820

cattcttgca gggtatggcg cgggcgtggc gggagctctt gtagcattca agatcatgag 5880

cggtgaggtc ccctccacgg aggacctggt caatctgctg cccgccatcc tctcgcctgg 5940

agcccttgta gtcggtgtgg tctgcgcagc aatactgcgc cggcacgttg gcccgggcga 6000

gggggcagtg caatggatga accggctaat agccttcgcc tcccggggga accatgtttc 6060

ccccacgcac tacgtgccgg agagcgatgc agccgcccgc gtcactgcca tactcagcag 6120

cctcactgta acccagctcc tgaggcgact gcatcagtgg ataagctcgg agtgtaccac 6180

tccatgctcc ggttcctggc taagggacat ctgggactgg atatgcgagg tgctgagcga 6240

ctttaagacc tggctgaaag ccaagctcat gccacaactg cctgggattc cctttgtgtc 6300

ctgccagcgc gggtataggg gggtctggcg aggagacggc attatgcaca ctcgctgcca 6360

ctgtggagct gagatcactg gacatgtcaa aaacgggacg atgaggatcg tcggtcctag 6420

gacctgcagg aacatgtgga gtgggacgtt ccccattaac gcctacacca cgggcccctg 6480

tactcccctt cctgcgccga actataagtt cgcgctgtgg agggtgtctg cagaggaata 6540

cgtggagata aggcgggtgg gggacttcca ctacgtatcg ggtatgacta ctgacaatct 6600

taaatgcccg tgccagatcc catcgcccga attcttcaca gaattggacg gggtgcgcct 6660

acacaggttt gcgccccctt gcaagccctt gctgcgggag gaggtatcat tcagagtagg 6720

actccacgag tacccggtgg ggtcgcaatt accttgcgag cccgaaccgg acgtagccgt 6780

gttgacgtcc atgctcactg atccctccca tataacagca gaggcggccg ggagaaggtt 6840

ggcgagaggg tcaccccctt ctatggccag ctcctcggct atccagctgt ccgctccatc 6900

tctcaaggca acttgcaccg ccaaccatga ctcccctgac gccgagctca tagaggctaa 6960

cctcctgtgg aggcaggaga tgggcggcaa catcaccagg gttgagtcag agaacaaagt 7020

ggtgattctg gactccttcg atccgcttgt ggcagaggag gatgagcggg aggtctccgt 7080

acctgcagaa attctgcgga agtctcggag attcgcccgg gccctgcccg tctgggcgcg 7140

gccggactac aaccccccgc tagtagagac gtggaaaaag cctgactacg aaccacctgt 7200

ggtccatggc tgcccgctac cacctccacg gtcccctcct gtgcctccgc ctcggaaaaa 7260

gcgtacggtg gtcctcaccg aatcaaccct atctactgcc ttggccgagc ttgccaccaa 7320

aagttttggc agctcctcaa cttccggcat tacgggcgac aatacgacaa catcctctga 7380

gcccgcccct tctggctgcc cccccgactc cgacgttgag tcctattctt ccatgccccc 7440

cctggagggg gagcctgggg atccggatct cagcgacggg tcatggtcga cggtcagtag 7500

tggggccgac acggaagatg cgatcgcgtg agcgcaacgc aattaatgtg agttagctca 7560

ctcattaggc accccaggct ttacacttta tgcttccggc tcgtatgttg tgtggaattg 7620

tgagcggata acaatttcac acaggaaaca gctatgacca tgattacgcc aagcttgcat 7680
gcctgcaggt cgactctaga ggatccccgg gtaccggtcg ccaccatggc tctttcaaac 7740
aagtttatcg gagatgacat gaaaatgacc taccatatgg atggctgtgt caatgggcat 7800
tactttaccg tcaaaggtga aggcagcggg aagccatacg aagggacgca gacctcgact 7860
tttaaagtca ccatggccaa cggtgggccc cttgcattct cctttgacat actatctaca 7920
gtgttcatgt atggaaatcg atgctttact gcgtatccta ccagtatgcc cgactatttc 7980
aaacaagcat ttcctgacgg aatgtcatat gaaaggactt ttacctatga agatggagga 8040
gttgctacag ccagttggga aataagcctt aaaggcaact gctttgagca caaatccacg 8100
tttcatggag tgaactttcc tgctgatgga cctgtgatgg cgaagatgac aactggttgg 8160
gacccatctt ttgagaaaat gactgtctgc gatggaatat tgaagggtga tgtcaccgcg 8220
ttcctcatgc tgcaaggagg tggcaattac agatgccaat tccacacttc ttacaagaca 8280
aaaaaaccgg tgacgatgcc accaaaccat gcggtggaac atcgcattgc gaggaccgac 8340
cttgacaaag gtggcaacag tgttcagctg acggagcacg ctgttgcaca tataacctct 8400
gttgtccctt tctgaagcgg ccgcgactct agaattccaa ctcggtccgg gtaaacactc 8460
cggcctctta agccatttcc tgtttttttt tttttttttt tttttttttt cttttttttt 8520
ttctttcctt tccttctttt tttcctttct ttttcccttc tttaatggtg gctccatctt 8580
agccctagtc acggctagct gtgaaaggtc cgtgagccgc ttgactgcag agagtgctga 8640
tactggcctc tctgcagatc aagtactagt agaggcggtt tgcgtattgg gcgctcttcc 8700
gcttcctcgc tcactgactc gctgcgctcg gtcgttcggc tgcggcgagc ggtatcagct 8760
cactcaaagg cggtaatacg gttatccaca gaatcagggg ataacgcagg aaagaacatg 8820
tgagcaaaag gccagcaaaa ggccaggaac cgtaaaaagg ccgcgttgct ggcgtttttc 8880
cataggctcc gcccccctga cgagcatcac aaaaatcgac gctcaagtca gaggtggcga 8940
aacccgacag gactataaag ataccaggcg tttccccctg gaagctccct cgtgcgctct 9000
cctgttccga ccctgccgct taccggatac ctgtccgcct ttctcccttc gggaagcgtg 9060
gcgctttctc atagctcacg ctgtaggtat ctcagttcgg tgtaggtcgt tcgctccaag 9120
ctgggctgtg tgcacgaacc ccccgttcag cccgaccgct gcgccttatc cggtaactat 9180
cgtcttgagt ccaacccggt aagacacgac ttatcgccac tggcagcagc cactggtaac 9240

aggattagca gagcgaggta tgtaggcggt gctacagagt tcttgaagtg gtggcctaac 9300

tacggctaca ctagaaggac agtatttggt atctgcgctc tgctgaagcc agttaccttc 9360

ggaaaaagag ttggtagctc ttgatccggc aaacaaacca ccgctggtag cggtggtttt 9420

tttgtttgca agcagcagat tacgcgcaga aaaaaaggat ctcaagaaga tcctttgatc 9480

ttttctacgg ggtctgacgc tcagtggaac gaaaactcac gttaagggat tttggtcatg 9540

agattatcaa aaaggatctt cacctagatc cttttaaatt aaaaatgaag ttttaaatca 9600

atctaaagta tatatgagta aacttggtct gacagttacc aatgcttaat cagtgaggca 9660

cctatctcag cgatctgtct atttcgttca tccatagttg cctgactccc cgtcgtgtag 9720

ataactacga tacgggaggg cttaccatct ggccccagtg ctgcaatgat accgcgagac 9780

ccacgctcac cggctccaga tttatcagca ataaaccagc cagccggaag ggccgagcgc 9840

agaagtggtc ctgcaacttt atccgcctcc atccagtcta ttaattgttg ccgggaagct 9900

agagtaagta gttcgccagt taatagtttg cgcaacgttg ttgccattgc tacaggcatc 9960

gtggtgtcac gctcgtcgtt tggtatggct tcattcagct ccggttccca acgatcaagg 10020

cgagttacat gatcccccat gttgtgcaaa aaagcggtta gctccttcgg tcctccgatc 10080

gttgtcagaa gtaagttggc cgcagtgtta tcactcatgg ttatggcagc actgcataat 10140

tctcttactg tcatgccatc cgtaagatgc ttttctgtga ctggtgagta ctcaaccaag 10200

tcattctgag aatagtgtat gcggcgaccg agttgctctt gcccggcgtc aatacgggat 10260

aataccgcgc cacatagcag aactttaaaa gtgctcatca ttggaaaacg ttcttcgggg 10320

cgaaaactct caaggatctt accgctgttg agatccagtt cgatgtaacc cactcgtgca 10380

cccaactgat cttcagcatc ttttactttc accagcgttt ctgggtgagc aaaaacagga 10440

aggcaaaatg ccgcaaaaaa gggaataagg gcgacacgga aatgttgaat actcatactc 10500

ttcctttttc aatattattg aagcatttat cagggttatt gtctcatgag cggatacata 10560

tttgaatgta tttagaaaaa taaacaaata ggggttccgc gcacatttcc ccgaaaagtg 10620

ccacctgacg tctaagaaac catt 10644

<210> 9
<211> 39
<212> DNA
<213> Artificial Sequence

<220>
<223>  AsiSI sense-strand primer for PCR amplification of patient NS5B

<400>  9
ggaggctagt gaggacgcga tcgcttgctc aatgtccta                              39


<210>  10
<211>  39
<212>  DNA
<213>  Artificial Sequence

<220>
<223>  AsiSI sense-strand primer for PCR amplification of patient NS5B

<400>  10
ggaggctagt gaggacgcga tcgcttgctc aatgtctta                              39


<210>  11
<211>  39
<212>  DNA
<213>  Artificial Sequence

<220>
<223>  AsiSI sense-strand primer for PCR amplification of patient NS5B


<220>
<221>  misc_feature
<222>  (31)
<223>  where r is a or g

<400>  11
ggaggctagt gaggacgcga tcgcttgctc ratgtccta                              39


<210>  12
<211>  39
<212>  DNA
<213>  Artificial Sequence

<220>
<223>  AsiSI sense-strand primer for PCR amplification of patient NS5B

<400>  12
ggaggctagt gaggacgcga tcgcctgctc aatgtctta                              39


<210>  13
<211>  39
<212>  DNA
<213>  Artificial Sequence

<220>
<223>  AsiSI sense-strand primer for PCR amplification of patient NS5B

<400>  13
ggaggctagt gaggacgcga tcgcctgctc aatgtccta                              39


<210>  14
<211>  39
<212>  DNA
<213>  Artificial Sequence

<220>
<223>  AsiSI sense-strand primer for PCR amplification of patient NS5B


<220>
<221>  misc_feature
<222>  (31)
<223>  where r is a or g

<400>  14
ggaggctagt gaggacgcga tcgcttgctc ratgtctta                              39


<210>  15
<211>  39
<212>  DNA
<213>  Artificial Sequence

<220>
<223>  AsiSI sense-strand primer for PCR amplification of patient NS5B


<220>
<221>  misc_feature
<222>  (25)
<223>  where y is c or t

<220>
<221>  misc_feature
<222>  (28)
<223>  where y is c or t

<400>  15
ggaggctagt gaggacgcga tcgcytgytc aatgtctta                              39


<210>  16
<211>  39
<212>  DNA
<213>  Artificial Sequence

<220>
<223>  AsiSI sense-strand primer for PCR amplification of patient NS5B

<400>  16
ggaggctagt gaggacgcga tcgcctgctc gatgtctta                          39


<210>  17
<211>  39
<212>  DNA
<213>  Artificial Sequence

<220>
<223>  AsiSI sense-strand primer for PCR amplification of patient NS5B

<400>  17
ggaggctagt gaggacgcga tcgcctgttc gatgtccta                          39


<210>  18
<211>  39
<212>  DNA
<213>  Artificial Sequence

<220>
<223>  AsiSI sense-strand primer for PCR amplification of patient NS5B


<220>
<221>  misc_feature
<222>  (31)
<223>  where r is a or g

<400>  18
ggaggctagt gaggacgcga tcgcctgctc ratgtccta                          39


<210>  19
<211>  39
<212>  DNA
<213>  Artificial Sequence

<220>
<223>  AsiSI sense-strand primer for PCR amplification of patient NS5B

<400>  19
ggaggctagt gaggacgcga tcgcctgctc gatgtccta                          39


<210>  20
<211>  39
<212>  DNA
<213>  Artificial Sequence

<220>
<223>  SacII antisense-strand primer for PCR amplification of patient
       NS5B

<400>  20
ggcctggagt gtttagctcc gcggtcatcg gttggggta                              39


<210>  21
<211>  39
<212>  DNA
<213>  Artificial Sequence

<220>
<223>  SacII antisense-strand primer for PCR amplification of patient
       NS5B


<220>
<221>  misc_feature
<222>  (28)
<223>  where y is c or t

<400>  21
ggcctggagt gtttagctcc gcggtcaycg gttggggag                              39


<210>  22
<211>  39
<212>  DNA
<213>  Artificial Sequence

<220>
<223>  SacII antisense-strand primer for PCR amplification of patient
       NS5B

<400>  22
ggcctggagt gtttagctcc gcggtcatcg gttgggaag                              39


<210>  23
<211>  39
<212>  DNA
<213>  Artificial Sequence

<220>
<223>  SacII antisense-strand primer for PCR amplification of patient
       NS5B

<400>  23
ggcctggagt gtttagctcc gcggtcaacg gttgggaag                              39


<210>  24

<211>  39
<212>  DNA
<213>  Artificial Sequence

<220>
<223>  SacII antisense-strand primer for PCR amplification of patient
       NS5B

<400>  24
ggcctggagt gtttagctcc gcggtcatcg gttgggaag                              39


<210>  25
<211>  40
<212>  DNA
<213>  Artificial Sequence

<220>
<223>  RsrII antisense-strand primer for PCR amplification of patient
       NS5B


<220>
<221>  misc_feature
<222>  (32)
<223>  where r is a or g

<400>  25
ggcctggagt gtttagctcg gaccgtcatc grttggggag                             40


<210>  26
<211>  40
<212>  DNA
<213>  Artificial Sequence

<220>
<223>  RsrII antisense-strand primer for PCR amplification of patient
       NS5B

<400>  26
ggcctggagt gtttagctcg gaccgtcacc ggttggggag                             40


<210>  27
<211>  40
<212>  DNA
<213>  Artificial Sequence

<220>
<223>  RsrII antisense-strand primer for PCR amplification of patient
       NS5B

<220>
<221>  misc_feature
<222>  (29)
<223>  where y is a or t

<400>  27
ggcctggagt gtttagctcg gaccgtcayc ggttggggag                              40


<210>  28
<211>  40
<212>  DNA
<213>  Artificial Sequence

<220>
<223>  RsrII antisense-strand primer for PCR amplification of patient
       NS5B

<400>  28
ggcctggagt gtttagctcg gaccgtcatc ggttgggaag                              40


<210>  29
<211>  40
<212>  DNA
<213>  Artificial Sequence

<220>
<223>  RsrII antisense-strand primer for PCR amplification of patient
       NS5B

<400>  29
ggcctggagt gtttagctcg gaccgtcaac ggttgggaag                              40


<210>  30
<211>  40
<212>  DNA
<213>  Artificial Sequence

<220>
<223>  RsrII antisense-strand primer for PCR amplification of patient
       NS5B

<400>  30
ggcctggagt gtttagctcg gaccgtcaac ggttggggta                              40


<210>  31
<211>  40
<212>  DNA
<213>  Artificial Sequence

<220>

<223>  RsrII antisense-strand primer for PCR amplification of patient
      NS5B

<220>
<221>  misc_feature
<222>  (38)
<223>  where r is a or g

<400>  31
ggcctggagt gtttagctcg gaccgtcatc ggttgggrag                              40

<210>  32
<211>  40
<212>  DNA
<213>  Artificial Sequence

<220>
<223>  RsrII antisense-strand primer for PCR amplification of patient
      NS5B

<400>  32
ggcctggagt gtttagctcg gaccgtcatc ggttggggta                              40

<210>  33
<211>  40
<212>  DNA
<213>  Artificial Sequence

<220>
<223>  RsrII antisense-strand primer for PCR amplification of patient
      NS5B

<400>  33
ggcctggagt gtttagctcg gaccgtcatc ggttgggaag                              40